# EUROPEAN PATENT APPLICATION

(11) **EP 4 435 097 A1**
(43) Date of publication of application: **25.09.2024**
(21) Application number: 23802658.7
(22) Date of filing: 26.04.2023
(51) Int. Cl.: C12N 9/10, C12N 15/54, C12N 15/74, C12N 15/75, C12N 15/81, C12N 15/80, C12P 19/26, C12P 19/18

(54) **POLYPEPTIDE HAVING GLYCOSIDE HYDROLASE ACTIVITY, PREPARATION METHOD THEREFOR, AND USE THEREOF**

(30) Priority: 13.05.2022 CN 202210517234
(71) Applicant: Shandong Henglu Biotech. Co., Ltd., Jinan, Shandong 250004 (CN)
(72) Inventor: HAN, Lijuan, Jinan, Shandong 250004 (CN); FANG, Xu, Jinan, Shandong 250004 (CN); NIU, Kangle, Jinan, Shandong 250004 (CN); TANG, Qi, Jinan, Shandong 250004 (CN); LI, Bin, Jinan, Shandong 250004 (CN); HIROSE, Yoshihiko, Ogaki Gifu 503-0097 (JP)
(74) Representative: LLR
(86) International application number: PCT/CN2023/090862
(87) International publication number: WO 2023/216885

(57) **Abstract**

The present application provides a polypeptide having glycoside hydrolase activity, a related nucleic acid product and enzyme product thereof, a preparation method therefor, and use thereof in synthesis of human milk oligosaccharides (HMOS), particularly lacto-N-biose (LNB). The polypeptide having glycoside hydrolase activity of the present application is used as a catalyst, and can efficiently catalyze the reaction of acetylglucosamine or a carbohydrate having an acetylglucosamine group with a glycosyl donor to generate the lacto-N-biose (LNB). Moreover, a process for producing the LNB based on the peptide is simple and convenient to operate and has a significant increase in production efficiency, thus being more suitable for industrial production.

## Description

### Cross Reference

The present application claims priority to Chinese Patent Application, filed on May 13, 2022, with the application number 202210517234.8 and the title "POLYPEPTIDE HAVING GLYCOSIDE HYDROLASE ACTIVITY, PREPARATION METHOD THEREFOR, AND USE THEREOF", which is incorporated herein by reference in its entirety.

### Technical Field

The present application belongs to the technical field of enzyme engineering/gene engineering, and specifically relates to a polypeptide having an activity of glycoside hydrolase, related nucleic acid products and enzyme products thereof, preparation method therefor, and use thereof.

### Background

Human milk oligosaccharides (HMOs), a type of complex oligosaccharides, are composed of monosaccharides and its derivatives, sialic acid, and other structural units linked by glycosidic bonds. However, it has taken more than a hundred years to be aware of them.

As early as 1886, an Australian pediatrician and microbiologist, Escherich discovered a correlation between the intestinal bacteria of infants and their digestive health. In 1900, two researchers, Moro and Tissier, independently confirmed that the bacterial flora in the excrement differed significantly between infants fed on breast milk and those on formula. Are there components in breast milk that cause differences in the gut bacteria of infants? In 1926, Schönfeld identified a whey component that stimulated Bifidobacterium bifidum growth, yet the precise nature of this component remained elusive until 1930 when French scientists Polonowski and Lespagnol discovered carbohydrate-like substances in breast milk whey and termed them "gynolactose." In 1954, György confirmed that the factor "Bifidus Factor" that promotes the growth of bifidobacteria is actually oligosaccharides. In the same year, two scientists, Polonowski and Montreuil, used two-dimensional chromatography to separate oligosaccharides from milk oligosaccharides. In 1983, Egge et al. confirmed HMOs using fast atom bombardment mass spectrometry. In 1999, Coppa et al. detected that 30-50% of HMOs remained structurally intact in the feces of breastfed babies. In 2000, some researchers discovered that HMOs resist enzymatic degradation within the infant gut, underscoring their role in biological functions beyond mere nutrition and energy.

At the dawn of the 21^{st} century, the enhanced research methodologies revealed a decline in the total concentration of HMOs, both acidic and neutral, as lactation progresses. Acknowledging the paramount importance of HMOs for infant development and long-term health, they are known to foster a balanced gut microecology, stimulate beneficial bacterial growth, curb pathogens, resist infectious diseases, diminish the risk of inflammatory bowel conditions and gastroenteritis, modulate the immune response, and support cognitive development in infants.

Breast milk is the only material and energy connection between a newborn and her/his mother, and is considered as the gold standard for infant nutrition. Human milk oligosaccharides (HMOs) are the third largest component in breast milk after lactose and lipids. They play an irreplaceable role in improving the digestive system, intestinal health and immune system of infants and young children. Human milk oligosaccharides (HMOs) are added to infant milk powder to simulate the components of breast milk, which is particularly important for the nutritional health of some infants who are unable to breastfeed and for post-lactation infants.

The European Commission issued Regulation (EU) No. 2020/484 on April 2, 2020, approving the marketing of Lacto-N-tetraose (LNT) as a novel food and listing it in the Authorization List of Novel Foods established according to Commission (EU) Regulation No. 2017/2470. This product was developed by Glycom A/S Company and produced by the fermentation process of the genetically modified *Escherichia coli*K12 DH1 strain. It is mainly used in sterilized dairy products, fermented milk products, cereal bars, flavored beverages, processed cereal foods, infant foods, etc. Due to high price and limited production, the said LNT has not been widespread. And, the limited sources and the difficulties in large-scale synthesis using chemical methods have restricted the research of its biological functions and widespread application.

Currently, infant formula milk powder, which is widely concerned by the public, all uses cow's milk or goat's milk as raw materials. But the cow's milk or goat's milk lacks most of the HMOs commonly found in human milk, and cannot achieve the nutrition, immunity, etc. of breast milk for infants and young children. Adding human milk oligosaccharides with physiological functions to infant milk powder, artificial milk, dairy products, infant food, functional health products or food for special groups will greatly improve the physical quality of the corresponding population as well as their quality of life, which has obvious social and economic value. Therefore, there is an urgent need for stable industrialized production of HMOs to supply market demand.

Although chemical methods can effectively produce oligosaccharides with defined structures, and many new strategies for synthesizing oligosaccharides have been developed in recent years, the synthesis of oligosaccharides with relatively complex structures still requires a large workload, and its yield is low. Besides that, the control of stereoselectivity and regioselectivity in the chemical synthesis of oligosaccharides is also a difficult problem. Lacto-N-biose (Galβ1-3GlcNAc, referred to as LNB, CAS number: 63121-25-5), with a chemical structural formula such as Formula I, is the most abundant component and the main common core structure of HMOs. Limited supply sources and difficulties in large-scale synthesis using chemical methods have restricted the research and widespread application of its biological functions. In order to break through this bottleneck, it is urgent to establish a simple, efficient, and economical synthesis route.

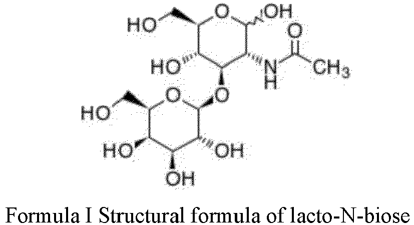

The Reference Patent 1 describes a method for synthesizing lacto-N-biose. The method includes: galactokinase and lacto-N-biose phosphohydrolase (LNBP) are added into a reaction system containing galactose, acetyl glucosamine and lactose as substrates to prepare the lacto-N-biose. In the reaction system above, acetyl phosphate, and acetokinase are also added to regenerate ATP in-situ, and the reaction is performed in *vitro.* The patent technique utilizes a multienzyme catalytic system that has favorable biosecurity and wide application, and introduces an ATP regeneration cycle system into the multienzyme reaction system, thereby enhancing the yield and decreasing the reaction time. The process of synthesizing HMOs using galactose-1-phosphate as a donor is simple and efficient, which not only provides a foundation for large-scale industrial production, but also has important economic value and social benefit. However, in this reaction system, the activity of Lnbp enzyme is inhibited by phosphorus ions generated in the reaction, thereby affecting the transformation efficiency.

The Reference Document 2 also shows that the synthesis efficiency of LNB is not high using only galactokinase and lacto-N-biose phosphorylase. In view of the technical bottlenecks such as the large demand for energy raw material ATP in the existing multi-enzyme synthesis process and the inhibited enzyme activity caused by the phosphorus ions generated in the existing ATP substrate cycle module, a large amount of pyruvate oxidase, acetate kinase and catalase are used to construct LNB synthesis module, ATP regeneration module, and PyoD-driven phosphate cycle module. A cycle among ATP, phosphate, hydrogen peroxide and oxygen forms an in-situ cofactor regeneration closed-loop system in the enzyme-linked reaction. By optimizing reaction conditions and controlling speed-limiting steps, the initial ATP concentration is reduced, and the yield of LNB is increased, which reaches 0.96 mmol LNB/mol GlcNAc in a 100-mL reaction system. Although the LNB transformation rate is increased and the initial ATP concentration is reduced by combining ATP regeneration and Pi alleviation, the complexity and the time of the process increase greatly due to the adding of pyruvate oxidase, acetate kinase and catalase; and further optimization is needed to realize large-scale production.

The Reference Document 3 records a multienzyme cascade system wherein sucrose and GlcNAc are used as substrates and four enzymes, namely sucrose phosphorylase, UDP-glucose hexose-1-uridine phosphate transferase, UDP glucose 4-epimerase and lactose-N-biological phosphorylase are used as catalyst. The yield of synthesized LNB reaches 83% when the reaction reaches 600 hours. The reaction time of this process is too long, which is not conducive to industrial production.

In order to meet the market demand and supply a large amount of LNB, it is necessary to develop an enzyme with a high transformation rate and a process for producing LNB to improve productivity and reduce production costs. It is believed that in the near future, through the unremitting efforts of biochemical researchers, HMOs will be widely used in dairy products, food, pharmaceuticals, cosmetics, and feed products, benefiting the public.

### Reference patents/documents:

1. Chinese invention patent, a method for synthesizing lacto-N-biose, patent authorization number ZL201911055516.5.
2. Du Z, Liu Z, Tan Y, Niu K, Guo W, Jia Y, Fang X (2021) Lacto-N-biose Synthesis via a Modular Enzymatic Cascade with ATP Regeneration. iScience 24(3):102236.
3. Nishimoto M, Kitaoka M (2007) Practical preparation of lacto-N-biose I, a candidate for the bifidus factor in human milk. Biosci Biotechnol Biochem 71(8): 2101-2104.

### Summary

### Objective

The present application aims to provide a polypeptide with glycoside hydrolase activity, which can be used to efficiently generate Lacto-N-biose (LNB). In addition, the present application also provides the said polypeptide-related nucleic acid product and enzyme product, and a preparation method and application of the polypeptide with glycoside hydrolase activity.

### Technical solutions

According to a Carbohydrate-Active-Enzymes (CAZy database) classification method, glycoside hydrolase (EC 2.4.1.211) with the amino acid sequence shown in SEQ ID NO: 1 belongs to GH112 glycoside hydrolase family, and it has a high ability of catalyzing β-1,3-Galactosyl-N-acetylhexosamine (GlcNAC) into LNB.

The glycoside hydrolase (EC 2.4.1.211) with the amino acid sequence shown in SEQ ID NO: 1 can be naturally derived from *Bacillus mycoides* (lnbp, Genebank number QWJ02347) or *Bacillus cereus* (lnbp, Genebank number WP_078422288.1).

The inventor of the present application performed a series of modifications on the glycoside hydrolase with the amino acid sequence shown in SEQ ID NO: 1 and obtained a polypeptide that also has glycoside hydrolase activity (namely, a glycoside hydrolase mutant), and the polypeptide had more excellent performance in the aspect of synthesizing lacto-N-biose (LNB).

Specifically, in a first aspect, the present application provides a polypeptide with glycoside hydrolase activity, which has an amino acid sequence selected from the follows:
an amino acid sequence having glycoside hydrolase activity, obtained by modifying, substituting, deleting, or adding one or more amino acids of the amino acid sequence shown in SEQ ID NO: 1;
wherein the modification, substitution, deletion, or addition of the one or more amino acids is selected from the follows:
   i) a combination of substitution from glutamic acid to glycine at position 229, substitution from valine to glycine at position 459, substitution from alanine to glycine at position 460, and substitution from alanine to leucine at position 271;
   ii) a combination of substitution from aspartic acid to asparagine at position 233, substitution from glycine to valine at position 339, substitution from threonine to phenylalanine at position 343, and substitution from valine to glycine at position 459;
   iii) a combination of substitution from aspartic acid to asparagine at position 233, substitution from alanine to leucine at position 271, substitution from glycinamide to serine at position 323, and substitution from glycine to valine at position 339;
   iv) a combination of substitution from glutamic acid to glycine at position 229, substitution from alanine to leucine at position 271, substitution from glycine to valine at position 339, and substitution from valine to glycine at position 459; and
   v) a combination of substitution from valine to glycine at position 459, and substitution from alanine to glycine at position 460.

In a second aspect, the present application provides a polynucleotide which encodes the polypeptide in the first aspect.

In a third aspect, the present application provides a nucleic acid construct which comprises the polynucleotide in the second aspect and one or more regulatory sequences operably linked to the polynucleotide, and the said regulatory sequences can guide the expression of the polypeptide in an appropriate host.

In a fourth aspect, the present application provides an expression vector which comprises the polynucleotide in the second aspect or comprises the nucleic acid construct in the third aspect.

In a fifth aspect, the present application provides a transformed host cell in which the nucleic acid construct in the third aspect or the expression vector in the fourth aspect is introduced.

Preferably, the host cell is any one type of bacteria, fungi, or yeast;
more preferably, the bacteria are selected from: *Escherichia coli, Vibrio natriegens, Bacillus subtilis, Bacillus licheniformis,* and *Bacillus amyloliquefaciens;*
more preferably, the fungi are selected from: *Aspergillus oryzae, Aspergillus niger, Penicillium oxalicum*, and *Trichoderma reesei;* and
more preferably, the yeast are selected from: *Pichia pastoris, Saccharomyces cerevisiae, Kluyveromyces lactis*, and *Kluyveromyces marxianus.*

In a sixth aspect, the present application provides an enzyme agent or an enzyme composition which contains the polypeptide in the first aspect and optionally other enzyme; and
preferably, the other enzyme is lactase and/or galactokinase.

In a seventh aspect, the present application provides a production method of the polypeptide in the first aspect. The production method includes:
(1) culturing a host cell comprising a nucleic acid construct under conditions conducive to the production of the polypeptide, wherein the nucleic acid construct comprises a nucleotide sequence encoding the polypeptide; and
(2) collecting the polypeptide.

In the production method above, the host cell is described in the fifth aspect.

In the specific embodiment, step (1) includes: firstly, the nucleic acid construct or a recombinant expression vector capable of encoding the polypeptide in the first aspect is introduced into the host cell to construct an engineered host cell capable of expressing the polypeptide; and then, the engineered host cells are cultured and induced to express the polypeptide.

In a practicable embodiment, the host cell is *Escherichia coli* or *Vibrio natriegens,* and in this case, the plasmid of the recombinant expression vector for transforming the host cell is pET-32a;
in a practicable embodiment, the host cell is *Bacillus subtilis*, and in this case, the plasmid of the recombinant expression vector for transforming the host cell is pEB03;
in a practicable embodiment, the host cell is *Pichia pastoris,* and in this case, the plasmid of the recombinant expression vector for transforming the host cell is pPIC9K;
in a practicable embodiment, when the host cell is *Kluyveromyces lactis*, in this case, the plasmid of the recombinant expression vector for transforming the host cell is pKLAC1; and
in a practicable embodiment, the host cell is *Kluyveromyces marxianus*, and in this case, the plasmid of the recombinant expression vector for transforming the host cell is pKD1.

In a specific embodiment, step (2) includes: after the said polypeptides are expressed in host cells, the cultured cells are fragmented to obtain a crude enzyme solution containing the polypeptide; and optionally, the steps of separating and purifying the polypeptide are further included.

Preferably, the conventional protein purification methods may be used in the said step of separating and purifying the polypeptide, such as ion exchange chromatography, hydrophobic chromatography, gel filtration chromatography, salting-out and the like or a combination thereof; and further preferably, Ni column affinity chromatography can be used to purify the polypeptide.

In an eighth aspect, the present application provides a method for producing lacto-N-biose (LNB), which includes: using a glycoside hydrolase with the amino acid sequence as shown in SEQ ID NO: 1 or a mutant thereof (namely the polypeptide with glycoside hydrolase activity as described in the first aspect) as a catalyst, acetylglucosamine or a carbohydrate comprising an acetylglucosamine group reacts with a glycosyl donor to generate lacto-N-biose;
wherein, the glycoside hydrolase with the amino acid sequence as shown in SEQ ID NO: 1 or the mutant thereof catalyzes acetylglucosamine (GlcNAC) to produce LNB according to the following reaction formula:

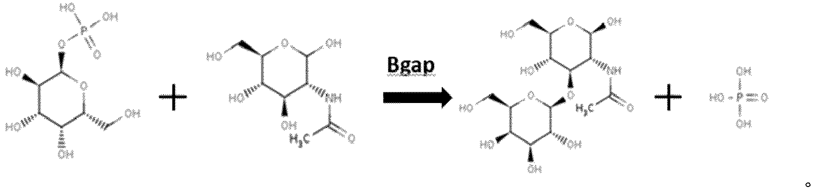

In the method above, preferably, the glycosyl donor is any one of or a combination of two or more of galactose-1-phosphate, lactose, and galactose;
when galactose-1-phosphate is used as a substrate, the reaction can be catalyzed by glycoside hydrolase or a mutant monoenzyme thereof;
when lactose is used as the substrate, a catalytic system of the reaction includes: lactase, the glycoside hydrolase or the mutant thereof, and galactokinase; and the transformation rate of GlcNAC is up to 94.69% in the absence of ATP circulation.

Preferably, the reaction is performed at 10-70°C, preferably 20-50°C, more preferably 30-50°C;
preferably, the reaction is performed in a buffer solution with pH 4-11, preferably PH 6-8; and more preferably, the buffer solution is selected from:
   a 3-morpholinopropanesulfonic acid (MOPS) buffer solution with a pH buffer range of 6.5-7.9;
   a 4-(2-Hydroxyethyl)-1-piperazineethanesulfonic acid (HEPES) buffer solution with a pH buffer range of 6.8-8.2;
   a tris(hydroxymethyl)aminomethane hydrochloride (Tris-HCl) buffer solution with a pH buffer range of 7.0-9.0; and
   a phosphate buffer solution with a pH buffer range of 5.8-8.0.

Preferably, the reaction solvent is a water-containing solvent or a solution containing solids.

In the above method for producing lacto-N-biose, a purified form of the glycoside hydrolase with the amino acid sequence shown in SEQ ID NO: 1 or the mutant thereof can be used, or the crude enzyme liquid of the glycoside hydrolase can also be used; and in addition, the glycoside hydrolase or the mutant thereof can be used after being immobilized, or mixed with other materials capable of maintaining or improving the activity of the glycoside hydrolase or the mutant thereof to serve as an enzyme preparation.

In a ninth aspect, the present application provides an application of glycoside hydrolase with the amino acid sequence shown in SEQ ID NO: 1 or the polypeptide in the first aspect as a catalyst in production of human milk oligosaccharides, especially lacto-N-biose;
it is to be noted that in the prior art, there have been no reports of using the glycoside hydrolase BgaP with the amino acid sequence shown in SEQ ID NO: 1 for the synthesis of LNB; and moreover, compared with the amino acid sequence of the lacto-N-biose phosphorylase (LNBP) as described in a Document "Lacto-N-biose Synthesis via a Modular Enzymatic Cascade with ATP Regeneration. iScience 24(3):102236", and the amino acid sequence of the lacto-N-biose phosphorylase BAE95374.1 lacto-N-biose phosphorylase 2 [Bifidobacterium bifidum JCM 1254] as described in the Document "Practical Preparation of Lacto-N-biose I, a Candidate for the Bifidus Factor in Human Milk", the amino acid sequence shown as the SEQ ID NO: 1 has less than 55% sequence similarity.

Preferably, the method for producing Lacto-N-biose is as described in the eighth aspect.

### Advantages:

According to the present application, the polypeptide (or glycoside hydrolase mutant) with glycoside hydrolase activity can catalyze acetylglucosamine and/or the carbohydrate with acetylglucosamine group to generate lacto-N-biose (LNB) at very high efficiency in the presence of the glycosyl donor, and the transformation rate of GlcNAC can reach 94.69% in absence of ATP circulation.

Besides, the present application also provides the method for producing lacto-N-biose (LNB). Compared with the production method related to a multienzyme or multienzyme-ATP circulation system in the prior art, the method according to the present application obviously enhances the transformation rate of GlcNAC, and it is simple to operate and suitable for industrial production, greatly decreases the reaction time by about 30 times as compared with 600 h in the prior art, and obviously enhances the production efficiency.

The technical solutions of the present application have positive meanings for industrial production of human milk oligosaccharides. The method is eco-friendly, efficient and sustainable, and beneficial to application of industrial large-scale production. According to the present application, the polypeptide with glycoside hydrolase activity and application thereof have very important economic and social values.

### Brief Description of the Drawings

FIG. 1. SDS-PAGE electrophoretogram of 1,3-β-galactosyl-N-acetylhexosamine phosphorylase Bgap (lane 1) as prepared in Example 1, mutants Bgap-8 (lane 2) and Bgap-11 (lane 3) as prepared in Example 9, and a reference protein substance (lane 4, kDa).
FIG. 2. HPLC diagram of LNB synthesized by the catalysis of Bgap, Bgap-8 and Bgap-11 respectively.
FIG. 3. LC-MS diagram of an LNB standard substance.
FIG. 4. LC-MS diagram of LNB synthesized by the catalysis of Bgap.
FIG. 5. LC-MS diagram of LNB synthesized by the catalysis of Bgap-8.
FIG. 6. LC-MS diagram of LNB synthesized by the catalysis of Bgap-11.

### Detailed Description

The experimental methods used in the following embodiments are conventional methods unless otherwise stated; and all materials, reagents, etc., unless otherwise stated, can be obtained from commercial sources.

The pET-32a plasmids were purchased from Sangon Biotech (Shanghai) Co., Ltd.

The present application will be described in more detail by the following embodiments. It is to be understood that the specific embodiments described here are only used to explain the present application and are not used for limiting the present application. Modifications or substitutions of the details and forms of the technical solution without departing from the structural ideas and scope of use of the present application shall fall within the protection scope of the present application.

Unless otherwise defined, all technical and scientific terms used in this specification have the same meanings as commonly understood by those skilled in the art. In general, the nomenclature used in this specification and the experimental methods described below are well known and commonly used in the art.

In the following embodiments and examples, the HPLC analysis conditions of the product obtained by the catalysis of glycoside hydrolase were as follows: chromatographic column type: Cosmosil Sugar-D amino column (nacalaitesque, INC.); the mobile phase was 75% acetonitrile aqueous solution; the temperature was 30°C; the injection volume was 10 µL; a UV detector (Hitachi Chromaster) was adopted; the wavelength was 210 nm; and the flow rate was 1.0 mL/min.

It is to be noted that the terms used herein are only for describing specific embodiments and are not intended to limit the exemplary embodiments according to the present application. Furthermore, it is also to be understood that when the terms "comprising" and/or "including" are used in this specification, they indicate the presence of features, steps, operations, devices, components and/or combinations thereof. It is to be understood that the protection scope of the present application is not limited to the following specific embodiments; it is also to be understood that the terms used in the embodiments of the present application are for describing specific embodiments rather than limiting the protection scope of the present application. In the following specific embodiments, if no specific experimental conditions are specified, the conventional methods and conditions of molecular biology in this technical field are usually followed. Such techniques and conditions are fully explained in the literature. See, for example, the techniques and conditions described in "Molecular Cloning: A Laboratory Manual" of Sambrook et al., or follow the conditions recommended by the manufacturer.

### Example 1. Preparation of wild type 1,3-β-galactosyl-N-acetylhexosamine phosphorylase and determination of its ability to catalyze the synthesis of LNB

The preservation numbers of *Bacillus mycoides* and *Bacillus cereus* in the ATCC (American Type Culture Collection) are: ATCC6462 and ATCC9634 respectively.

*Bacillus mycoides*/*Bacillus cereus* (glycerol stock) were correspondingly inoculated on LB plates (LB culture medium: 1.0% of peptone, 0.5% of yeast extract, and 1.0% of NaCl; for plates 1.5% of agar powder was added and dissolved), and cultured in an incubator at 37°C overnight. Single colonies were picked up and put into 3 mL of LB culture medium, and cultured at 37°C and 200 rpm for 6-8 h; 2.5 mL of culture reaction was inoculated into 50 mL of fermentation culture medium (1% of tryptone, 0.5% of yeast powder, and 1.0% of NaCl), and subjected to shaking culture at 34°C and 250 rpm for 72 h; and then thallus was collected. The thallus was resuspended in 50 mM of PBS with pH of 7.4, and disrupted with an AH-BASIC high-pressure homogenizer until the system was homogenized. Centrifuging was performed at 13,000 rpm for 30 min; then the supernatant was collected, and filtered with a 0.45 µm water-based polyethersulfone membrane; the supernatant was separated with a HiPrepTM16/60 SephacrylTMS-300HR molecular sieve using 50 mM of PBS buffer solution with pH of 7.4 as a mobile phase at a flow rate of 0.5 mL/min; the component at the peak with the molecular weight of about 83-84 kDa was collected; and then concentrated by ultrafiltration through a 10 kDa ultrafiltration tube; and the obtained supernatant contained the 1,3-β-galactosyl-N-acetylhexosamine phosphorylase (Bgap enzyme) shown as SEQ ID NO: 1.

The ability of 1,3-β-galactosyl-N-acetylhexosamine phosphorylase to catalyze the synthesis of LNB was determined:
32 mM of galactose-1-phosphate (Gal-1-P), 20 mM ofN-acetylglucosamine (GlcNAc), 32 mM of ATP, 0.3 mg/mL of the enzyme with the amino acid sequence shown in SEQ ID NO: 1, 50 mM of phosphate, and 100 mM of tris(hydroxymethyl)aminomethane hydrochloride (Tri-HCl) buffer solution were uniformly mixed; and the pH value was 7.0; the reaction was terminated after the reaction was performed at 37°C for 20 h. Purification was carried out by a gel column method; and the concentration of LNB in the purified solution detected by HPLC and was 0.73 mg/mL, and the transformation rate of GlcNAc was 9.57%.

### Example 2. Expression of wild type 1,3-β-galactosyl-N-acetylhexosamine phosphorylase in Escherichia coli, and determination of its ability to catalyze the synthesis of LNB

According to a method in "Molecular Cloning: A Laboratory Manual", PCR amplification was performed by taking *Bacillus mycoides* genome DNA as a template (the gene encoding 1,3-β-galactosyl-N-acetylhexosamine phosphorylase shown as SEQ ID NO: 1 was temporarily named as *bgap*)*,* designing an upstream primer as ATCGGATCCGAATTCAAAAAGAAAAAAGGCCGTGTTACTTTACCGAGT (SEQ ID NO: 3) and a downstream primer as TGCGGCCGCAAGCTTGCCTTCATTTGAAACATC (SEQ ID NO: 4), so as to obtain a target gene segment having the size of 2,172 bp. Agarose gel electrophoresis was performed for the PCR product; a target gene *bgap* was recovered with a gel recovery kit; and then enzyme digestion was performed on the target gene *bgap* and a plasmid pET-32a with two restriction enzymes (*EcoR* I and *Hind* III). An enzyme digestion system was as follows: 2 µL of target gene Bgap or 2 µL of plasmid pET-32a, 2 µL of *EcoR* I, 2 µL of *Hind* III, and 5 µL of 10x enzyme digestion buffer solution, and deionized water added to reach 50 µL; and enzyme digestion was performed at 37°C. The product was subjected to agarose gel electrophoresis, and then a target band on gel was recovered by the kit. The target gene subjected to enzyme digestion was connected into a vector by a DNA ligase Ligation high, and reacted in a water bath for 30 min to obtain a recombinant expression plasmid pET32a-Bgap.

The recombinant plasmid which was verified to be correct was transformed into *Escherichia coli* BL21(DE3) according to the following steps: the prepared *Escherichia coli* BL21(DE3) competent cells were placed on ice for 30 min for thawing; 100 µL of competent cells were mixed with 10 µL of pET32a-Bgap recombinant plasmid (concentration of 50 ng/µL); the mixture were placed in a water bath at 42°C for heat shock for 45s, and then immediately transferred into an ice bath for 2 min for cooling; 1 mL of fresh LB culture medium (LB culture medium: 1.0% of peptone, 0.5% of yeast extract, and 1.0% of NaCl; for plates 1.5% of agar powder was added and dissolved) was added to perform recovery culture for 1 h at 37°C and 100 rpm; then 100 µL of thallus liquid was coated onto LB plates containing ampicillin (100 µg /mL) and cultivate for 12 h in a constant-temperature incubator at 37°C; then single colonies were picked up and subjected to recombinant identification by PCR and double enzyme digestion so as to verify whether the construction of the pET32a-Bgap recombinant plasmid was successfully introduced or not.

Then, positive transformants on the plates were picked up, inoculated into an LB liquid culture medium, and subjected to shaking culture on a shaking table at 37°C and 200 rpm for 12 h to obtain a seed solution; the seed solution was inoculated into a fresh LB culture medium according to an inoculation amount of 1% (v/v), subjected to shaking culture at 37°C until OD₆₀₀ was 0.8, and then induced by isopropyl-β-D-thiogalactopyranoside (IPTG) with a final concentration of 0.1 mM at 16°C and 200 rpm for 12 h. After induced expression, fermentation liquid was centrifuged at 4°C and 5000 r/min for 30 mins, and the thallus was collected. The thallus was resuspended into 20 mM of PBS buffer solution with pH of 7.4, and subjected to ultrasonic treatment at a frequency of plus on 5s/off 5s for 30 min to disrupt the thallus; and the disrupted liquid was centrifuged at 13,000 × g and 4°C for 30 mins to remove cell debris, and then the supernatant was collected.

Soluble lacto-N-biose phosphorylase was obtained through purification by nickel column affinity chromatography, and the process included: deionized water was added to reach the top end of a nickel column to perform naturally eluting, and then the column was eluted by 5 times the volume of Binding buffer; a crude enzyme solution filtered by a 0.45 µm filter membrane was loaded onto the column at a flow rate of 1.5 mL/min to enable a sufficient binding of the sample to the nickel column; continuous and gradient elution was performed by 5 times the volume of Washing buffer after the sample was drained so as to remove impure proteins, followed by eluting with 5 times the volume of Elution buffer and collecting the eluent to obtain the target protein. The expression condition of the target protein was expressed by SDS-PAGE. The genetic engineering bacteria had obvious specific expression bands after being induced, the molecular weight of the bands was basically consistent with the expected molecular weight of 102 kD, and the obtained protein was 1,3-β-galactosyl-N-acetylhexosamine phosphorylase Bgap shown in SEQ ID NO: 1.

The ability of 1,3-β-galactosyl-N-acetylhexosamine phosphorylase to catalyze the synthesis of LNB was determined:
32 mM of galactose-1-phosphate (Gal-1-P), 20 mM ofN-acetylglucosamine (GlcNAc), 32 mM of ATP, 0.3 mg/mL of the enzyme with the amino acid sequence shown in SEQ ID NO: 1, 50 mM of phosphate, and 100 mM of tris(hydroxymethyl)aminomethane hydrochloride (Tri-HCl) buffer solution were uniformly mixed; the pH value was 7.0; the reaction was terminated after the reaction was performed at 37°C for 20 h; purification was performed by a gel column method; and the concentration of LNB in the purified solution was detected by HPLC and was 2.11 mg/mL, and the transformation rate of GlcNAc was 27.54%.

### Example 3. Expression of wild type 1,3-β-galactosyl-N-acetylhexosamine phosphorylase in Vibrio natriegens: NBRC 15636, and determination of its ability to catalyze the synthesis of LNB

The acquisition of *Vibrio natriegens* competent cells included the following steps: a single colony of *Vibrio natriegens* was picked up from a newly activated plate, and inoculated into 5 mL of LB liquid culture medium (10 g/L of tryptone; 5 g/L of yeast extract; and 10 g/L of NaCl) to perform shaking culture at 37°C for about 12 h until reaching a logarithmic phase; the obtained bacterial suspension was transferred into 100 mL of LB liquid culture medium according to a ratio of 1: 100-1: 200, and cultured overnight; the culture was allowed to grow at 37°C with shaking at 200 rpm until OD₆₀₀ was 0.5; then the obtained culture solutions was added into two 250 mL of centrifuge bottles, respectively, which were then placed on ice for 15 min for cooling; the obtained culture solution was centrifuged at 4°C and 6,500 rpm for 20 min; the supernatant was removed, and the thallus was gently resuspended into 5-10 mL of an electrotransfer buffer solution (680 mM of sucrose, and 7 mM of K₂PO₄, and pH of 7), with blowing and mixing well; the thallus solution was centrifuged at 4°C and 6,750 rpm for 15 min; the obtained supernatant was washed 3 times by a pipette; the obtained cells were resuspended into the remaining electrotransfer buffer solution after the last cleaning; and OD₆₀₀ was adjusted to 16 with the electrotransfer buffer solution to obtain the *Vibrio natriegens* competent cells.

1 µL of the recombinant plasmid pET32a-Bgap (50 ng/µL) in the Example 1 and 100 µL of competent cells were gentally mixed in a 1.5 mL precooled centrifuge tube. The mixed solution was transferred into a 1 mm electrotransformation cup on a sterile operating table, placed on ice for 1 min, and then quickly placed into an electrotransformation instrument for electric shock at electrotransformation parameters of 800-900V, 25 µF and 200 ohm for 0.6 ms. After electric excitation, 500 µL of recovery culture medium (v2 salt (204 mM of NaCl, 4.2 mM of KCl and 23.14 mM of MgCl₂) was added into a BHI culture medium) was added, and transferred into a 15 mL culture tube. The incubation was continuously performed at 30-37°C for 1-2 h. The recovery culture solution was coated onto a hot LB agar plate containing corresponding antibiotics, and incubated at 37°C overnight until colonies appeared. The colonies were picked up and put into a TBv2 culture medium (12 g/L of tryptone, 24 g/L of yeast extract, 0.5% v/v of glycerol, 15 g/L of NaCl, 2.31 g/L of KH₂PO₄, and 16.43 g/L of K₂HPO₄·3H₂O) and subjected to enlarge cultivation by a shaking table at 30°C. After obtaining positive transformants of *Vibrio natriegens,* the protein purification method according to Example 1 was carried out, and the obtained protein was 1,3-β-galactosyl-N-acetylhexosamine phosphorylase with the amino acid sequence shown in SEQ ID NO: 1.

The ability of 1,3-β-galactosyl-N-acetylhexosamine phosphorylase to catalyze the synthesis of LNB was determined:
32 mM of galactose-1-phosphate (Gal-1-P), 20 mM ofN-acetylglucosamine (GlcNAc), 32 mM of ATP, 0.3 mg/mL of the enzyme with the amino acid sequence shown in SEQ ID NO: 1, 50 mM of phosphate, and 100 mM of tris(hydroxymethyl)aminomethane hydrochloride (Tri-HCl) buffer solution were uniformly mixed, respectively; the pH value was 7.0; the reaction was performed at 37°C for 20 h, respectively; then the reaction was terminated; purification was performed by a gel column method; and the concentration of LNB in the purified solution was detected by HPLC and was 0.78 mg/mL, and the transformation rate of GlcNAc was 10.24%.

### Example 4. Expression of wild type 1,3-β-galactosyl-N-acetylhexosamine phosphorylase in Bacillus subtilis 168, and determination of its ability to catalyze the synthesis of LNB

According to the gene sequence in the Example 1, an upstream primer was designed as GATAAGCTTATGAAAAAGAAAAAAGGCCGTGTTACTTTACCGAGTGAA (SEQ ID NO: 5), and a downstream primer was designed as CAGGAATTCGCCTTCATTTGAAACATCCTCCC (SEQ ID NO: 6). 50 µL of a PCR reaction system was as follows: 1 µL (about 50 ng) of a DNA template (total DNA of *Bacillus mycoides*)*,* 25 µL of 2 × Phanta Max Buffer, 1 µL of 10 pmol/µL dNTP Mix, 2 µL of 10 pmol/ µL forward primer, 2 µL of 10 pmol/ µL reverse primer, 1 µL of 1 U/µLPhanta Max Super-Fidelity DNA Polymerase, and ultrapure water added to reach 50 µL. PCR reaction process was as follows: 95°C for 3 min; 95°C for 15 s, 58°C for 15 s, 72°C for 2.5 min for 30 cycles; PCR reaction at 72°C for 5 min; and storing at 4°C. The PCR segment and pEB03 plasmid were respectively subjected to double digestion by restriction endonucleases *EcoR* I and *Hind* III, then connected by a T4 ligase, transformed into *Escherichia coli* DH5α, and subjected to screening and identification to obtain a recombinant plasmid pEB03-Bgap.

A method for transforming the pEB03-Bgap plasmid into *Bacillus subtilis* was as follows: a full cycle of *Bacillus subtilis* glycerides was inoculated onto an LB plate (an LB culture medium: 1.0% of peptone, 0.5% of yeast extract, and 1.0% of NaCl; for plates 1.5% of agar powder was added and dissolved), and cultured in an incubator at 37°C overnight; single colonies were picked up and put into 1 mL of LB culture medium and cultured at 37°C and 200 rpm overnight; 200 µL of the culture solution was transferred into 200 mL of growth culture medium (growth culture medium: 0.5 M sorbitol added into the LB culture medium), and cultured at 37°C and 200 rpm until OD₆₀₀ reached 0.85-0.95; the culture was then placed in ice bath for 10 min, followed by centrifuging at 4°C and 5,000 rpm for 5 min; the thallus was washed for 4 times with a precooled electrotransformation culture medium (electrotransformation culture medium: 0.5 M of sorbitol and 10% of glycerin added into the LB culture medium), and finally resuspended into 2 mL of precooled electrotransformation culture medium; the above treated bacterial solution was subpackaged into tubes at 80 µL each tube, to which 1 µL (about 50 ng) of pEB03-Bgap plasmid was added; the mixture was transferred into a precooled electrotransformation cup, and then cooled on ice for 1-1.5 min, which was followed by an electrotransformation by an electro transformation instrument under the following electrotransformation parameters: 2,000 V of voltage, and 200 Ω of resistance; the resultant was placed in ice bath for 2 min, and then 1 mL of recovery culture medium (recovery culture medium: 0.5 M of sorbitol and 0.38 M of mannitol added into the LB culture medium) was added to culture at 37°C and 200 rpm for 90 min; the bacterial solution was coated onto a LB plate containing chloramphenicol (5 µg/mL), and transformants were picked up to obtain the genetically engineered *Bacillus subtilis* strain.

Positive transformed single colonies were picked from the LB flat plate containing 5 µg/mL of chloramphenicol, inoculated into 50 mL of seed culture medium (0.5% of yeast extract powder, 0.5% of tryptone, 1% of glucose, 1.8% of K₂HPO₄, and 5 µg/mL of chloramphenicol), and subjected to shaking culture at 34°C and 210 rpm for 8 h. 2.5 mL of the obtained product was inoculated into 50 mL of fermentation culture medium (1.0% of tryptone, 0.5% of yeast powder, and 1.0% of NaCl), and subjected to shaking culture at 34°C and 250 rpm for 48 h; and SDS-PAGE electrophoresis detection was performed for the supernatant, and the obtained protein was 1,3-β-galactosyl-N-acetylhexosamine phosphorylase Bgap with the amino acid sequence shown in SEQ ID NO: 1.

The ability of 1,3-β-galactosyl-N-acetylhexosamine phosphorylase to catalyze the synthesis of LNB was determined:
32 mM of galactose-1-phosphate (Gal-1-P), 20 mM ofN-acetylglucosamine (GlcNAc), 32 mM of ATP, 0.3 mg/mL of the enzyme with the amino acid sequence shown in SEQ ID NO: 1, 50 mM of phosphate, and 100 mM of tris(hydroxymethyl)aminomethane hydrochloride (Tri-HCl) buffer solution were uniformly mixed, respectively; the pH value was 7.0; the reaction was terminated after the reaction was performed at 37°C for 20 h; purification was performed by a gel column method; and the concentration of LNB in the purified solution detected by HPLC and was 0.76 mg/mL, and the transformation rate of GlcNAc was 9.98%.

### Example 5. Expression of wild type 1,3-β-galactosyl-N-acetylhexosamine phosphorylase in Pichia pastoris: X33, and determination of its ability to catalyze the synthesis of LNB

According to a gene sequence optimized by a Bgap yeast codon provided by Sangon Biotech (Shanghai) Co., Ltd., an upstream primer was designed as GAAGCTTACGTAATGACTTCTACTGGTAGATTCAC (SEQ ID NO: 7), and a downstream primer was designed as TCAAGGCCTTAATTAAGCGGCCGCTTAGTGGTGATGGTGGTGATGCAAATTTC (SEQ ID NO: 8). 50 µL of a PCR reaction system was as follows: 1 µL of a DNA template, 25 µL of 2 x Phanta Max Buffer, 1 µL of 10 pmol/µL dNTP Mix, 2 µL of 10 pmol/µL forward primer, 2 µL of 10 pmol/µL reverse primer, 1 µL of 1 U/µLPhanta Max Super-Fidelity DNA Polymerase, and ddH₂O added to reach 50 µL. PCR reaction process was as follows: 95°C for 3 min; 95°C for 15 s, 60°C for 15s, 72°C for 1.5 min for 33 cycles; 72°C for 5 min; and stored at 4°C. The PCR segment and pPIC9K plasmid segment were respectively subjected to double digestion by restriction endonucleases *Snab*1 and *Not*I*,* then connected by a ligation high ligase, transformed into *Escherichia coli* DH5α, and subjected to ampicillin resistance screening to obtain a recombinant plasmid pPIC9K-Bgap.

Then, a PIC9K-Bgap expression cassette was amplified by the following steps: the upstream primer and the downstream primer were designed as GACGCAGATCGGGAACACTGAAAAAT (SEQ ID NO: 9) and ACTTTGATTTCTCTGGATTCGGAATGGTTTGAACG as (SEQ ID NO: 10), respectively, according to the sequence of pPIC9K plasmid. 50 µL of a PCR reaction system was as follows: 1 µL (about 50 ng) of a DNA template, 25 µL of 2 x Phanta Max Buffer, 1 µL of 10 pmol/µL dNTP Mix, 2 µL of 10 pmol/µL forward primer, 2 µL of 10 pmol/µL reverse primer, 1 µL of 1 U/µL Phanta Max Super-Fidelity DNA Polymerase, and ddH₂O added to reach 50 µL. PCR reaction process was as follows: 95°C for 3 min; 95°C for 15 s, 58°C for 15 s, 72°C for 5 min for 33 cycles; 72°C for 5 min; and stored at 4°C. Agarose gel electrophoresis was carried out, and then gel cutting and recycling were carried out to obtain the PIC9K-Bgap expression cassette.

The PIC9K-Bgap expression cassette was transformed into *Pichia pastoris* by a procedure including the following steps:
a single colony of freshly cultured *Pichia pastoris* was picked up and put into 1 mL of YPD culture medium, and cultured in a constant-temperature oscillator at 30°C and 250 rpm for 12-14 h; the culture was transferred into 50 mL of fresh YPD culture medium at initial inoculation OD of 0.2, and cultured at 30°C and 200 rpm for 3-4 h until OD was 0.8-1; the thallus culture was placed on ice for 5-10 min; at the same time, 25 µL of salmon sperm DNA (2 mg/mL) was heated at 95°C for 10 min by a PCR instrument, followed by immediately cooling in an ice water bath; and the cooled salmon sperm DNA was placed on ice for later use; a solution of Bgap expression cassette to be transformed was prepared, 5-10 ug of the solution was added into each tube, and supplemented to 50 µL with sterilized ddH₂O; the culture solution standing on ice was centrifuged at 4°C and 5,000x g for 5 min, the supernatant was removed, and the thallus was collected; the collected thallus was resuspended in sterilized ddH₂O, the suspension was subjected to centrifuging at 4°C and 5,000x g for 5 min, the supernatant was removed, and the thallus was collected; the operation was repeated once; 1 mL of 0.1 M LiCl solution was added to resuspend the thallus, the resuspended thallus was transferred to a new 1.5 mL centrifuge tube to perform centrifuging at 4°C and 13,000 x g for 15 s, and then the supernatant was removed; 400 µL of 0.1 M LiCl solution was added to resuspend the thallus, and the resuspended thallus was subpackaged into tubes at 50 µL each tube, and then centrifuged at 4°C and 13,000 x g for 15 s; the supernatant was removed, and the thallus was placed on ice for later use; 240 µL of 50% PEG3350 solution, 36 µL of 1M LiCl solution, 25 µL of the pretreated salmon sperm DNA solution, and 50 µL of Bgap expression cassette solution to be transformed were added into each tube, and uniformly mixed by violently whirling until the thallus was completely distributed (about 5 min); the uniformly mixed system was placed in a water bath at 30°C for 30 min, then placed in a constant-temperature water bath kettle at 42°C to perform heat shock for 25 min; the system was then allowed to cool to room temperature, followed by centrifuging at 8,000 x g for 2 min; the thallus was collected and resuspended in 1 mL of YPD, followed by shaking culture at 30°C; and 25-100 µL of thallus solution was coated onto a YPD culture plate with G418 geneticin resistance; and the plate was placed in a constant-temperature incubator at 30°C to perform an inverted culture for 2-3 days; and transformants were picked up to verify whether the transformation cassette had been successfully transformed.

Fermentation in Shaking Flask:
1) Single colonies were picked up, inoculated into a 100 mL of partition shake flask containing 10 mL of BMGY, and cultured at 28-30°C and 250-300 rpm until the OD₆₀₀ was 2-6 (about 16-18 h); 2) 10 mL of culture medium was inoculated into a 2 L of shake flask containing 1 L of BMGY, and vigorously oscillated at 28-30°C (250-300 rpm) until reaching the logarithmic phase (OD₆₀₀ was 2-6); 3) in the inducible expression process, the supernatant was discard, and the cells were resuspended in 1/5-1/10 original-volume of BMGY culture medium (100-200 mL); 4) the culture medium was added into a 1 L of partition shake flask, covered with 2 layers of sterile gauze or cheese wrapping cloth, and then transferred in a shaking table for continuing culturing at 28-30°C; 5) methanol was added at every 24 h to reach the concentration of 0.5%, and then the optimal induction time was reached; and 6) centrifuging was carried out at 1,500-3,000 g at room temperature for 5 min, and the cells were collected. The supernatant was discarded after intracellular expression, and the cells were immediately treated or stored at -80°C. The expression was secreted, the supernatant was reserved, and precooled at 4°C and can be concentrated if necessary. The protein was purified according to the method in the Example 1, or the supernatant was stored at -80°C for later use. The obtained protein was 1,3-β-galactosyl-N-acetylhexosamine phosphorylase Bgap shown as SEQ ID NO: 1.

The ability of 1,3-β-galactosyl-N-acetylhexosamine phosphorylase to catalyze the synthesis of LNB was determined:
32 mM of galactose-1-phosphate (Gal-1-P), 20 mM ofN-acetylglucosamine (GlcNAc), 32 mM of ATP, 0.3 mg/mL of enzyme shown as SEQ ID NO: 1, 50 mM of phosphate, and 100 mM of tris(hydroxymethyl)aminomethane hydrochloride (Tri-HCl) buffer solution were uniformly mixed, respectively; the pH value was 7.0; the reaction was terminated after the reaction was performed at 37°C for 20 h; purification was performed by a gel column method; and the concentration of LNB in the purified solution was detected by HPLC and was 0.66 mg/mL, and the transformation rate of GlcNAc was 8.67%.

### Example 6. Expression of wild type 1,3-β-galactosyl-N-acetylhexosamine phosphorylase in Kluyveromyces lactis (ATCC 12426), and determination of its ability to catalyze the synthesis of LNB

The construction method of recombinant plasmids pklac1-Bgap was shown as follows:
According to a gene sequence optimized by a Bgap yeast codon provided by a gene company, an upstream primer was designed as AGCTAGAAGAGCTAGATCTCCTAGGATGACTTCTACTGGTAGATTCACTTTGC (SEQ ID NO: 11), and a downstream primer was designed as TCAAGGCCTTAATTAAGCGGCCGCTTAGTGGTGATGGTGGTGATGCAAA (SEQ ID NO: 12). 50 µL of a PCR reaction system: 1 µL of a DNA template (plasmid provided by the gene company), 25 µL of 2 × Phanta Max Buffer, 1 µL of 10 pmol/µL dNTP Mix, 2 µL of 10 pmol/µL forward primer, 2 µL of 10 pmol/µL reverse primer, 1 µL of 1 U/ µLPhanta Max Super-Fidelity DNA Polymerase, and ddH₂O added to reach 50 µL. PCR reaction process was as as follows: 95°C for 3 min; 95°C for 15 s, 60°C for 15 s, at 72°C for 1.5 min for 33 cycles; 72°C for 5 min; and stored at 4°C. The PCR segment and plasmid segment were respectively subjected to double digestion by restriction endonucleases *Xmaj*I and *Not*I*,* then connected by a ligation high ligase, transformed into *Escherichia coli* DH5α, and subjected to screening and identifying to obtain a recombinant plasmid pklac1-Bgap.

Then, a klac1-Bgap expression cassette was amplified; according to the sequence of pklac1 plasmid, an upstream primer was designed as CCGCGGGGATCGACTCATAAAATAGTAACCTTCT (SEQ ID NO: 13) and a downstream primer GCCGCGGAAATTTAGGAATTTTAAACTTGGGCTTG as (SEQ ID NO: 14) was designed. 50 µL of a PCR reaction system: 1 µL (about 50 ng) of a DNA template (pklac1-Bgap plasmid), 25 µL of 2 x Phanta Max Buffer, 1 µL of 10 pmol/µL dNTP Mix, 2 µL of 10 pmol/µL forward primer, 2 µL of 10 pmol/µL reverse primer, 1 µL of 1 U/µL Phanta Max Super-Fidelity DNA Polymerase, and ddH₂O added to reach 50 µL. PCR reaction process was as follows: 95°C for 3 min; 95°C for 15 s, 60°C for 15 s, 72°C for 5 min for 33 cycles; 72°C for 5 min; and stored at 4°C. Agarose gel electrophoresis was carried out, and then gel cutting and recycling were carried out to obtain the klac1-Bgap expression cassette.

The klacl-Bgap expression cassette was transformed into *Kluyveromyces lactis* by a procedure including the following specific steps: a single colony of freshly cultured *Kluyveromyces lactis* was picked up and put into 1 mL of YPD culture medium, and cultured in a constant-temperature oscillator at 30°C and 250 rpm for 12-14 h; the culture was transferred into 50 mL of fresh YPD culture medium at initial inoculation OD of 0.2, and cultured at 30°C and 200 rpm for 3-4 h until OD was 0.8-1; the thallus culture was placed on ice for 5-10 min; meanwhile, 25 µL of salmon sperm DNA (2 mg/mL) was heated at 95°C for 10 min by a PCR instrument, followed by immediately cooling in an ice water bath; and the cooled salmon sperm DNA was placed on ice for later use; an expression cassette solution to be transformed was prepared, 5-10 ug of the solution was added into each tube, and supplemented to 50 µL with sterilized ddH₂O; the culture solution standing on ice was centrifuged at 4°C and 5,000 × g for 5 min, the supernatant was removed, and the thallus was collected; the collected thallus was resuspended in sterilized ddH₂O, the suspension was subjected to centrifuging at 4°C and 5,000 × g for 5 min, the supernatant was removed, and the thallus was collected; the operation was repeated once; 1 mL of 0.1 M LiCl solution was added to resuspend the thallus, the resuspended thallus was transferred to a new 1.5 mL centrifuge tube to perform centrifuging at 4°C and 13,000 × g for 15 s, and then the supernatant was removed; 400 µL of 0.1 M LiCl solution was added to resuspend the thallus, and the resuspended thallus was subpackaged into tubes at 50 µL each tube, and then centrifuged at 4°C and 13,000 × g for 15 s; the supernatant was removed, and the thallus was placed on ice for later use; 240 µL of 50% PEG3350 solution, 36 µL of 1M LiCl solution, 25 µL of the pretreated salmon sperm DNA solution, and 50 µL of expression cassette solution to be transformed was added into each tube, and uniformly mixed by violently whirling until the thallus was completely distributed (about 5 min); the uniformly mixed system was placed in a water bath at 30°C for 30 min, then placed in a constant-temperature water bath kettle at 42°C to perform heat shock for 25 min; the system was then allowed to cool to room temperature, followed by centrifuging at 8,000 × g for 2 min; the thallus was collected and resuspended in 1 mL of YPD, followed by shaking culture at 30°C for 1-4 h; 25-100 µL of thallus solution was coated onto a culture plate with G418 resistance; and the plate was placed in a constant-temperature incubator at 30°C, to perform an inverted culture for 2-3 d; and transformants were picked up to verify whether the transformation cassette had been successfully transformed.

Fermentation in Shaking Flask: 1) Preparation of a first-class strain: a single colony of the genetically engineered yeast strain was cultured overnight in 10 mL of YGD liquid culture medium at 30°C and 200 rpm, so as to obtain the first-class strain. 2) Preparation of a second-class strain: the first-class strain was inoculated into a 1 L of YGD liquid culture medium, and cultured at 30°C and 200 rpm until the OD₆₀₀ was about 7. 3) The YGD culture was centrifuged to obtain a crude enzyme solution, which was purified according to the method in Example 1, to obtain the target protein which was 1,3-β-galactosyl-N-acetylhexosamine phosphorylase Bgap with the amino acid sequence shown in SEQ ID NO: 1.

The ability of 1,3-β-galactosyl-N-acetylhexosamine phosphorylase to catalyze the synthesis of LNB was determined:
32 mM of galactose-1-phosphate (Gal-1-P), 20 mM ofN-acetylglucosamine (GlcNAc), 32 mM of ATP, 0.3 mg/mL of the enzyme with the amino acid sequence shown in SEQ ID NO: 1, 50 mM of phosphate, and 100 mM of tris(hydroxymethyl)aminomethane hydrochloride (Tri-HCl) buffer solution were uniformly mixed; the pH value was 7.0; the reaction was performed at 37°C for 20 h and then terminated; purification was performed by a gel column method; and the concentration of LNB in the purified solution was detected by HPLC and was 0.55 mg/mL, and the transformation rate of GlcNAc was 7.23%.

### Example 7. Expression of wild type 1,3-β-galactosyl-N-acetylhexosamine phosphorylase in Kluyveromyces marxianus (ATCC 36907), and determination of its ability to catalyze the synthesis of LNB

The construction of a Bgap expression cassette included the following steps: 1) amplification of a Bgap segment. According to a gene sequence optimized by Bgap yeast codon provided by a gene company, an upstream primer was designed as CAATTACAAGAGAACTTCTACTGGTAGATTCACTTTGCCATCTG (SEQ ID NO: 15), and a downstream primer was designed as ATGGTGGTGATGCAAATTTCTCCAAGCAATAGCAGATTGATC (SEQ ID NO: 16). 50 µL of a PCR reaction system: 1 µL of a DNA template (plasmids provided by the gene company), 25 µL of 2 × Phanta Max Buffer, 1 µL of 10 pmol/µL dNTP Mix, 2 µL of 10 pmol/µL forward primer, 2 µL of 10 pmol/µL reverse primer, 1 µL of 1 U/ µLPhanta Max Super-Fidelity DNA Polymerase, and ddH₂O added to reach 50 µL. PCR reaction process was as follows: 95°C for 3 min; 95°C for 15 s, 60°C for 15 s, 72°C for 1.5 min for 33 cycles; 72°C for 5 min; and stored at 4°C. 2) Amplification of an inulase promoter segment and a signal peptide segment. A genome was extracted from Kluyveromyces marxianus, and an upstream primer CAAACCGAAATGGGGCGTTGTTACCCCAG (SEQ ID NO: 17) and a downstream primer ACCAGTAGAAGTTCTCTTGTAATTGATAACTGAAGCACTG (SEQ ID NO: 18) were designed. 50 µL of a PCR reaction system: 1 µL of a DNA template (Kluyveromyces marxianus genome), 25 µL of 2 × Phanta Max Buffer, 1 µL of 10 pmol/µL dNTP Mix, 2 µL of 10 pmol/µL forward primer, 2 µL of 10 pmol/µL reverse primer, 1 µL of 1 U/ µLPhanta Max Super-Fidelity DNA Polymerase, and ddH₂O added to reach 50 µL. PCR reaction process was as follows: 95°C for 3 min; 95°C for 15 s, 60°C for 15 s, 72°C for 1 min for 33 cycles; 72°C for 5 min; and stored at 4°C. 3) Amplification of a terminator fragment. A genome was extracted from Kluyveromyces marxianus, and an upstream primer CAATTACAAGAGAACTTCTACTGGTAGATTCACTTTGCCATCTG (SEQ ID NO: 19) and a downstream primer CCTCCATGTCCCGGTAGAGGTGTGGTCAAT (SEQ ID NO: 20) were designed. 50 µL of a PCR reaction system: 1 µL of a DNA template, 25 µL of 2 × Phanta Max Buffer, 1 µL of 10 pmol/µL dNTP Mix, 2 µL of 10 pmol/µL forward primer, 2 µL of 10 pmol/µL reverse primer, 1 µL of 1 U/ µLPhanta Max Super-Fidelity DNA Polymerase, and ddH₂O added to reach 50 µL. PCR reaction process was as follows: 95°C for 3 min; 95°C for 15 s, 60°C for 15 s, 72°C for 1 min for 33 cycles; 72°C for 5 min; and stored at 4°C. 4) Amplification of a G418 resistance gene. An upstream primer CCTCTACCGGGACATGGAGGCCCAGAATAC (SEQ ID NO: 21) and a downstream primer AAGCAGATCAGACAGTATAGCGACCAGCATTC (SEQ ID NO: 21) were designed. 50 µL of a PCR reaction system: 1 µL of a DNA template, 25 µL of 2 × Phanta Max Buffer, 1 µL of 10 pmol/µL dNTP Mix, 2 µL of 10 pmol/µL forward primer, 2 µL of 10 pmol/µL reverse primer, 1 µL of 1 U/ µLPhanta Max Super-Fidelity DNA Polymerase, and ddH₂O added to reach 50 µL. PCR reaction process was as follows: 95°C for 3 min; 95°C for 15 s, 60°C for 15 s, 72°C for 1.5 min for 33 cycles; 72°C for 5 min; and stored at 4°C. 5) Amplification of an inulase terminator. An upstream primer TCGCTATACTGTCTGATCTGCTTACTTTACTAACGAC (SEQ ID NO: 23) and a downstream primer TCGTGGCAGCAATTCATGATCGTAACGTGGTA (SEQ ID NO: 24) were designed. 50 µL of a PCR reaction system: 1 µL of a DNA template, 25 µL of 2 × Phanta Max Buffer, 1 µL of 10 pmol/µL dNTP Mix, 2 µL of 10 pmol/µL forward primer, 2 µL of 10 pmol/µL reverse primer, 1 µL of 1 U/ µL Phanta Max Super-Fidelity DNA Polymerase, and ddH₂O added to reach 50 µL. PCR reaction process was as follows: 95°C for 3 min; 95°C for 15 s, 60°C for 15 s, 72°C for 1.5 min for 33 cycles; 72°C for 5 min; and stored at 4°C. 6) Connection of PCR fragments. ABclonal was connected with the G418 gene and the inulase terminator gene. PCR fusion was performed to connect an inulase gene promoter signal peptide, a Bgap gene and a terminator gene. PCR fusion was carried out to connect the fragments to obtain the Bgap expression cassette.

Then, the obtained Bgap expression cassette was converted into Kluyveromyces marxianus. The specific operation was as follows: a single colony of freshly cultured Kluyveromyces marxianus was picked up and put into 1 mL of YPD culture medium, and cultured in a constant-temperature oscillator at 30°C and 250 rpm for 12-14 h; the culture was transferred into 50 mL of fresh YPD culture medium at initial inoculation OD of 0.2, and cultured at 30°C and 200 rpm for 3-4 h until OD was 0.8-1; the thallus culture was placed on ice for 5-10 min; meanwhile, 25 µL of salmon sperm DNA (2 mg/mL) was heated at 95°C for 10 min by a PCR instrument, followed by immediately cooling in an ice water bath; and the cooled salmon sperm DNA was placed on ice for later use; an expression cassette solution to be transformed was prepared, 5-10 ug of the solution was added into each tube, and supplemented to 50 µL with sterilized ddH₂O; the culture solution standing on ice was centrifuged at 4°C and 5,000 × g for 5 min, the supernatant was removed, and the thallus was collected; the collected thallus was resuspended in sterilized ddH₂O, the suspension was subjected to centrifuging at 4°C and 5,000 × g for 5 min, the supernatant was removed, the thallus was collected; the above operation was repeated once; 1 mL of 0.1 M LiCl solution was added to resuspend the thallus, the resuspended thallus was transferred to a new 1.5 mL centrifuge tube to perform centrifuging at 4°C and 13,000 × g for 15 s, and the supernatant was removed; 400 µL of 0.1 M LiCl solution was added to resuspend the thallus, and the resuspended thallus was subpackaged into tubes at 50 µL each tube, and then centrifuged at 4°C and 13,000 × g for 15 s; the supernatant was removed, and the thallus was placed on ice for later use; 240 µL of 50% PEG3350 solution, 36 µL of 1M LiCl solution, 25 µL of the pretreated salmon sperm DNA solution, and 50 µL of expression cassette solution to be transformed were added into each tube, and uniformly mixed by violently whirling until the thallus was completely distributed (about 5 min); the uniformly mixed system was placed in a water bath at 30°C for 30 min, then placed in a constant-temperature water bath kettle at 42°C to perform heat shock for 25 min; the system was then allowed to cool to room temperature, followed by centrifuging at 8,000 × g for 2 min; the thallus was collected and resuspended in 1 mL of YPD, followed by shaking culture at 30°C for 1-4 h; 25-100 µL of thallus solution was coated onto a culture plate with G418 resistance; and the plate was placed in a constant-temperature incubator at 30°C, to perform an inverted culture for 2-3 d; and transformants were picked up to verify whether the transformation cassette had been successfully transformed.

Finally, a positive transformant was selected to produce lacto-N-biose phosphorylase. The operation steps included: 1) Preparation of a first-class strain: a single colony of the genetically engineered yeast strain was cultured overnight in 10 mL of YGD liquid culture medium at 30°C and 200 rpm, so as to obtain the first-class strain. 2) Preparation of a second-class strain: the first-class strain was inoculated into a 1 L of YGD, 5 g of inulin was added as an inducer, and culturing was performed in the G418 liquid culture medium at 30°C and 200 rpm until the OD₆₀₀ was about 7.3) The YGD culture was centrifuged to obtain a crude enzyme solution, which was purified according to the method in Example 1, to obtained the target protein which was 1,3-P-galactosyl-N-acetylhexosamine phosphorylase Bgap with the amino acid sequence shown in SEQ ID NO: 1.

The ability of 1,3-β-galactosyl-N-acetylhexosamine phosphorylase to catalyze the synthesis of LNB was determined:
32 mM of galactose-1-phosphate (Gal-1-P), 20 mM ofN-acetylglucosamine (GlcNAc), 32 mM of ATP, 0.3 mg/mL of the enzyme with the amino acid sequence shown in SEQ ID NO: 1, 50 mM of phosphate, and 100 mM of tris(hydroxymethyl)aminomethane hydrochloride (Tri-HCl) buffer solution were uniformly mixed, respectively; the pH value was 7.0; the reaction was performed at 37°C for 20 h and then terminated; purification was performed by a gel column method; and the concentration of LNB in the purified solution was detected by HPLC and was 0.5 mg/mL, and the transformation rate of GlcNAc was 6.57%.

### Example 8. Expression of wild type 1,3-β-galactosyl-N-acetylhexosamine phosphorylase in Aspergillus oryzae (ATCC 10124), and determination of its ability to catalyze the synthesis of LNB

According to the experiment, genome DNA was extracted from *Aspergillus oryzae*; an *Aspergillus oryzae* amylase promoter and a glycosidase terminator were amplified by taking the *Aspergillus oryzae* genome DNA as a template; the promoter, the terminator and an expression vector skeleton pCAMBIA1304 were subjected to enzyme digestion connection, respectively; the promoter PamyB and the terminator TagdA were connected into corresponding enzyme digestion sites, thus an *Aspergillus oryzae* reading frame was constructed, and a plasmid pCAMBIA01 was obtained; for *Bgap* gene and the plasmid pCAMBIA01, two sequences subjected to enzyme digestion with the same restriction enzyme were connected by *Sal*I and *Spe*I double enzyme digestion; the connected product was transformed into an *Escherichia coli* competent cell DH5α; positive clones were screened, and subjected to amplification culture; the plasmid pCAMBIA02 was extracted; sequencing verification was performed on the recombinant plasmid; and the sequencing result showed that the target gene was correct.

Preparation of competent *Agrobacterium tumefaciens* EHA105: 1) *Agrobacterium tumefaciens* EHA105 activated on a solid YEB plate was inoculated into 20 mL of liquid YEB culture medium, rifampicin antibiotics were added into the culture medium. Shaking culture was performed at 28°C and 180 rpm for 24 h. 2) *Agrobacterium tumefaciens* were inoculated into 50 mL of liquid YEB culture medium added with rifampicin according to the inoculation amount of 1%, and shaking culture was performed at 28°C and 180 rpm untill OD₆₀₀ was 0.8 for use. 3) 50 mL of thallus liquid was placed on ice for 30 min, and then centrifuged at 4°C and 5,000 rpm for 10 min, and then supernatant was discard. 4) 10 mL of precooled CaCl₂ solution with the concentration of 0.1 M was added; the thallus was resuspended, and then placed on ice for 30 min; and centrifuged at 4°C and 5,000 rpm for 10 min, and then the supernatant was discarded. 5) 2 mL of precooled CaCl₂ with the concentration of 0.1 M and 2 mL of glycerol were added to resuspend EHA105 cells; and every 100 µL cells are packaged once for use; and the cells were stored in an ultralow-temperature refrigerator at -70°C for later use.

The pCAMBIA02 plasmid was transformed into competent EHA105 by a freeze-thaw method: *1) Agrobacterium tumefaciens* competent EHA105 was thawed on ice, 10 µL of recombinant plasmid pCAMBIA02 was added, uniformly mixed, and then placed on ice for 30 min. 2) The mixed solution was quickly put into liquid nitrogen for cold stimulation for 5 min, then immediately put into a 28°C constant-temperature water bath kettle, and incubated for 5 min. 3) The mixed solution was placed on ice for 5 min. 4) 400 µL of rifampicin-free YEB liquid culture medium was added into the mixed solution, and subjected to shaking culture at 28°C and 180 rpm for 2 h. 5) 200 mL of bacterial solution was coated in a solid YEB plate containing rifampicin and kanamycin, and subjected to inverted culture at 28°C for 48 h to observe the formation of the colonies. 6) Single colonies were picked up and put into 20 mL of rifampicin-and kanamycin-containing YEB liquid culture medium, and subjected to shaking culture at 28°C and 180 rpm for 48 h. The colonies were subjected to PCR to identify and screen the positive clones. The plasmids DNA were extracted from the positive clone to for plasmid PCR detection.

*Aspergillus oryzae* was transformed by an *Agrobacterium tumefaciens-mediated* expression vector: 1) *Agrobacterium tumefaciens* EHA105 thallus liquid containing different expression vectors was inoculated into a liquid LB culture medium added with rifampicin and kanamycin respectively, and cultured at 28°C and 180 rpm for 24 h. 2) 2 mL of thallus liquid was added into a mixed culture medium consisting of 10 mL of liquid LB culture medium and 10 mL of basic culture medium, and subjected to shaking culture at 28°C and 180 rpm for 48 h. 3) 2 mL of thallus liquid was added into 20 mL of induction culture medium, subjected to shaking culture at 28°C and 180 rpm, and then co-cultured with the *Aspergillus oryzae* when OD₆₀₀ was 0.8. 4) *Aspergillus oryzae* conidium suspension was prepared. 5) 100 µL of conidium suspension and 100 µL of EHA105 thallus liquid were uniformly mixed, and then coated on a solid induction culture medium paved with a microporous filter membrane; acetosyringone was added into the induction culture medium, and co-cultured at 28°C for 3 d. 6) The microporous filter membrane was uncovered, reversely paved on a Czapek's medium containing hygromycin B and cephalosporin, and then subjected to antibiotic screening culture at 30°C for 3 d. 7) The microporous filter membrane was removed; culturing was continued until resistant bacterial colonies grew; the screened resistant bacterial colonies were transferred to another selective culture medium, and subjected to continuous screening culture. The selected resistant transformants were verified by genomic PCR.

Expression of Bgap protein: the engineered *Aspergillus oryzae* strain as constructed were activated, cultured at a constant temperature of 30°C for 3 d, and washed with sterile water to obtain spores, so as to obtain a bacterial suspension for inoculation. Different strains were respectively inoculated into a solid fermentation culture medium according to a ratio of 2%, further cultured at 30°C and 200 rpm for 6 d by an inducible expression culture medium, and then centrifuged at 12,000 rpm; the supernatant was collected, and concentrated with a 3 kDa ultrafiltration membrane. Finally, nickel column affinity chromatography according to Example 1 was carried out, and the obtained protein was 1,3-β-galactosyl-N-acetylhexosamine phosphorylase Bgap with the amino acid sequence shown in SEQ ID NO: 1.

The ability of 1,3-β-galactosyl-N-acetylhexosamine phosphorylase to catalyze the synthesis of LNB was determined:
32 mM of galactose-1-phosphate (Gal-1-P), 20 mM ofN-acetylglucosamine (GlcNAc), 32 mM of ATP, 0.3 mg/mL of the enzyme with the amino acid sequence shown in SEQ ID NO: 1, 50 mM of phosphate, and 100 mM of tris(hydroxymethyl)aminomethane hydrochloride (Tri-HCl) buffer solution were uniformly mixed, respectively; the pH value was 7.0; the reaction was performed at 37°C for 20 h and then terminated; purification was performed by a gel column method; and the concentration of LNB in the purified solution was detected by HPLC and was 0.44 mg/mL, and the transformation rate of GlcNAc was 5.76%.

### Example 9. Preparation of glycoside hydrolase mutants

Bgap with an amino acid sequence shown in SEQ ID NO: 1 was modified by the protein engineering technology combined with high-throughput screening, so as to obtain a series of mutants that remarkably improved the catalytic synthesis efficiency of LNB. The specific steps included:
based on the homology and conserved sequence of the amino acid sequence of GH112 family glycoside hydrolase, the homologous modeling of Bgap was performed, and some sites were predicted by computer-aided rational design, such as Glu229, Asp233, Ala271, Gly323, Gly339, Thr343, Val459 and Ala460, which possibly affected the synthesis of LNB by the Bgap. Degenerate primers (shown as Table 1) were designed for the sites according to the gene sequence (shown as SEQ ID NO: 2) of the Bgap.

**Table 1**

| Amino acid site | Degenerate primers and DNA sequences | |
|---|---|---|
| 229 (Glu) | F | ACAACCTGGGCAAANNNAAATTTGTGGACT (SEQ ID NO: 25) |
| | R | AGTCCACAAATTTNNNTTTGCCCAGGTTGT (SEQ ID NO: 26) |
| 233 (Asp) | F | CAAAGAAAAATTTGTGNNNTGGTTTGGCTAGGCGC (SEQ ID NO: 27) |
| | R | GCGCCTAGCCAAACCANNNCACAAATTTTTCTTTG (SEQ ID NO: 28) |
| 271 (Ala) | F | ATCAGGGCTATTATAACACCNNNTTTCGCATTCCGACCCC (SEQ ID NO: 29) |
| | R | GGGGTCGGAATGCGAAANNNGGTGTTATAATAGCCCTGAT (SEQ ID NO: 30) |
| | F | TGGCACCGAACCGTATNNNAAATATTTTGAAAACA (SEQ ID NO: 31) |
| | R | TGTTTTCAAAATATTTNNNATACGGTTCGGTGCCA (SEQ ID NO: 32) |
| 339 (Gly) | F | TGGTGGGCAGCGTGNNNGGTGGTGCGACTTTA (SEQ ID NO: 33) |
| | R | TAAAGTCGCACCACCNNNCACGCTGCCCACCA (SEQ ID NO: 34) |
| 343 (Thr) | F | CGTGGGTGGTGGTGCGNNNTTACGTATGATTGCGGA (SEQ ID NO: 35) |
| | R | TCCGCAATCATACGTAANNNCGCACCACCACCCACG (SEQ ID NO: 36) |
| 459 (Val) | F | TGGCAGACCCATATGNNNGCGATGCGCTGTGGTAT (SEQ ID NO: 37) |
| | R | ATACCACAGCGCATCGCNNNCATATGGGTCTGCCA (SEQ ID NO: 38) |
| 460 (Ala) | F | CGCAGACCCATATGGTGNNNCATGCGCTGTGGTATAA (SEQ ID NO: 39) |
| | R | TTATACCACAGCGCATGNNNCACCATATGGGTCTGCG (SEQ ID NO: 40) |

Site-specific saturation mutation was carried out on these sites by overlap-PCR to obtain a series of mutant fragments; an expression vector was constructed according to the method described in Example 1 and transformed to *Escherichia coli,* and the transformants were respectively inoculated into a 96-well plate containing an LB culture medium, and then were subjected to shaking culture at 37°C for 16 h; shaking culture was continued at 16°C for 18 h after IPTG (the final concentration was 0.1 mM) was added; and then fermentation was finished. Lysozyme (the final concentration was 1 mg/mL) was added into the 96-well plate, and shaken at 37°C for 1 h to obtain a cell lysis solution; magnetic beads were added to adsorb Bgap proteins with CBM markers; the magnetic beads were recovered to obtain the purified Bgap. The efficiency of Bgap catalytic synthesis of LNB was determined according to the method in the Example 3. The Bgap mutants with different transformation efficiencies were obtained by high-throughput screening, and the corresponding transformants were sequenced to determine the mutation sites and sequences.

A large amount of target proteins were prepared from transformants that contain corresponding mutants of the sites Glu229, Asp233, Ala271, Gly323, Gly339, Thr343, Val459 and Ala460 according to the method in Example 1, and the efficiency of catalytic synthesis of LNB was repeatedly measured. Finally, favorable mutation was determined.

The results of detection showed that single-point substitution of Glu229Gly, Asp233Asn, Ala271Leu, Gly323Ser, Gly339Val, Thr343Phe, Val459Gly and Ala460Gly all contributed to improvement of the efficiency of synthesizing LNB. The single-point substitution was combined and optimized to obtain the mutants of protease shown in SEQ ID NO: 1. The details are shown in Table 2.

**Table 2. Bgap mutants and mutation patterns**

| Original enzymes or mutants | Mutation patterns |
|---|---|
| Bgap original enzyme | / |
| Bgap-1 | E229G/A271L/V459G/A460G |
| Bgap-2 | A271L/G323S/T343F/V459G |
| Bgap-3 | D233N/A271L/G339V |
| Bgap-4 | D233N/A271L/V459G |
| Bgap-5 | E229G/D233N/A460G |
| Bgap-6 | D233N/G339V/T343F/V459G |
| Bgap-7 | E229G/G323S/T343F |
| Bgap-8 | D233N/A271L/G323S/G339V |
| Bgap-9 | G339V/T343F/A460G |
| Bgap-10 | E229G/A271L/G339V/V459G |
| Bgap-11 | V459G/A460G |
| Bgap-12 | V459G |
| Bgap-13 | V459A |

### Example 10. Identification of glycoside hydrolase and mutants thereof

1,3-β-galactosyl-N-acetylhexosamine phosphorylase obtained in Example 1 and the glycoside hydrolase mutants obtained in Example 9 were analyzed and identified through TLC chromatography.

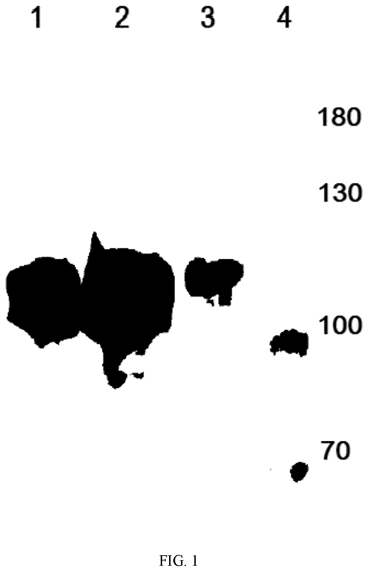

The TLC analysis method was as follows:
TLC analysis was carried out by using a TLC plate (Merck Kieselgel 60 F254) produced by Merck Company and taking a solution composed of N-butyl alcohol, absolute ethyl alcohol and ultrapure water by a ratio of 5:3:2 as a developing solvent; and after the TLC plate was dried, it was to check spots on the TLC plate through a UV lamp. The details are shown in FIG. 1.

As shown in FIG. 1, the purified 1,3-β-galactosyl-N-acetylhexosamine phosphorylase Bgap obtained in Example 1 is on the lane 1, the glycoside hydrolase mutant Bgap-8 obtained in Example 9 is on the lane 2, the glycoside hydrolase mutant Bgap-11 obtained in Example 9 is on the lane 3, and a contrast is on the lane 4.

As shown in FIG. 1, the molecular weight of the Bgap protein is about 83 kDa; the molecular weight of Bgap fusion protein expressed by using pET32a as a vector is about 102 kDa (containing Trx-tag), and the molecular weights of the Bgap-8 fusion protein and the Bgap-11 fusion protein are basically consistent with that of the Bgap.

In addition, according to the same method, other glycoside hydrolase mutants obtained in Example 9 were also identified (the result was not shown), and the molecular weights of the other glycoside hydrolase mutants were basically consistent with that of the Bgap.

### Example 11. Catalyzing the synthesis of LNB by taking a glycoside hydrolase or mutants thereof as catalysts, and galactose-1-phosphate as glycosyl donor

The efficiency of catalytic synthesis of LNB by the identified glycoside hydrolase Bgap and the mutant thereof in Example 10 was detected by using galactose-1-phosphate as the glycosyl donor. The specific method was as follows:
the reaction system included 32 mM of galactose-1-phosphate, 20 mM of N-acetylglucosamine, 0.3 mg/mL of glycoside hydrolase or mutants thereof, and 100 mM of Tris-HCl buffer solution; and the pH was 7.0. The reaction system continuously reacted at 37°C for 16 h, and then the reaction was terminated; the reaction system was purified by a gel column method; HPLC was used for detecting the concentration of LNB in the purified solution, the transformation rate was calculated. The result is shown in Table 3.

**Table 3. Research on synthesis of LNB catalyzed by a glycoside hydrolase or mutants thereof**

| Original enzyme or mutant | Generated LNB (mg/mL) | GlcNAc transformation rate (%) | Productivity (mg/mL /h) |
|---|---|---|---|
| Bgap | 2.11 | 27.54 | 0.13 |
| **Bgap-1** | **7.26** | **94.69** | **0.45** |
| Bgap-2 | 0.42 | 5.42 | 0.03 |
| Bgap-3 | 0.69 | 9.00 | 0.04 |
| Bgap-4 | 1.05 | 13.55 | 0.07 |
| Bgap-5 | 1.15 | 14.84 | 0.07 |
| **Bgap-6** | **6.3** | **82.17** | **0.39** |
| Bgap-7 | 1.55 | 20.24 | 0.10 |
| **Bgap-8** | **7.23** | **94.30** | **0.45** |
| Bgap-9 | 0.89 | 11.62 | 0.06 |
| **Bgap-10** | **6.57** | **85.69** | **0.41** |
| **Bgap-11** | **7.14** | **93.12** | **0.45** |
| Bgap-12 | 0.42 | 5.42 | 0.03 |
| Bgap-13 | 0.69 | 9.0 | 0.04 |

Results: the enzymes with high efficiency of catalyzing GlcNAc to generate LNB were mutants Bgap-1, Bgap-6, Bgap-8, Bgap-10 and Bgap-11 among a series of mutants of glycoside hydrolase Bgap, and the transformation rates of catalyzing GlcNAc to generate LNB by using them were all above 80%, and some can reach above 94% to the maximum, which were far higher than the GlcNAc transformation rate (27.54%) of the original enzyme Bgap.

### Example 12. Identification of synthetic products in Example 11

HPLC and LC-MS methods are used to identify the product LNB that obtained by glycoside hydrolase or mutants thereof catalyzing galactose-1-phosphate to react with N-acetylglucosamine in Example 11.

Specifically, HPLC detection and/or mass spectrometric detection were/was used for LNB standard substance and reaction products obtained by Bgap, Bgap-8 and Bgap-11 enzymes catalyzing galactose-1-phosphate (Gal-1-P) to react with N-acetylglucosamine in Example 11 respectively.

### (1) HPLC analysis

The HPLC analyzer and analysis conditions were shown as above.

The LNB standard substance was purchased from an agent Seebio Biotech (Shanghai) Co, Ltd., and the original product was produced by French ELICITYL Company.

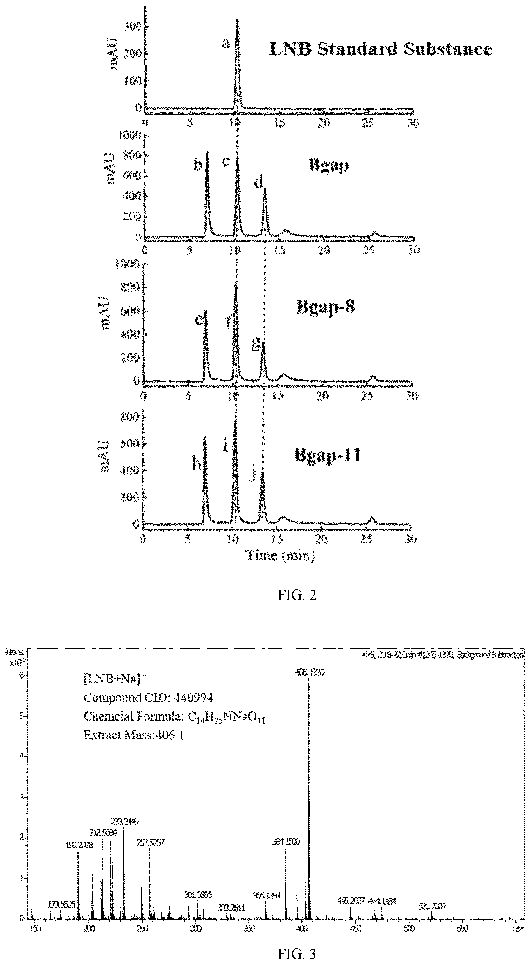

The HPLC analysis result showed that the retention time of the LNB standard substance was 10.3 min, as shown in FIG. 2.

The HPLC analysis result also showed that the reaction products obtained by Bgap, Bgap-8 and Bgap-11 enzymes catalyzing galactose-1-phosphate (Gal-1-P) to react with N-acetylglucosamine respectively had a strong absorption peak at 10.3 min, and the peak was consistent with the retention time of the LNB standard substance, as shown in FIG. 2.

### (2) LC-MS analysis

The substances with retention time of 10.3 min (corresponding to c peak, f peak, and i peak in HPLC spectra, respectively) in the catalytic products of Bgap, Bgap-8 and Bgap-11 enzymes were analyzed by LC-MS. The conditions for carrying out LC-MS analysis were the same as HPLC conditions, specifically, the model of a chromatographic column was Aminex HPX-87H (Bio-Rad, Inc., Heracles.), a detector was a Hitachi Chromaster), the detection wavelength was 210 nm, the sample size was 10 µL, the flow velocity was 0.5 ml/min, the column temperature was 45°C, a mobile phase was a 5 mM sulfuric acid aqueous solution, and the scanning range of molecular weight in an H-ESI mode was 400-900.

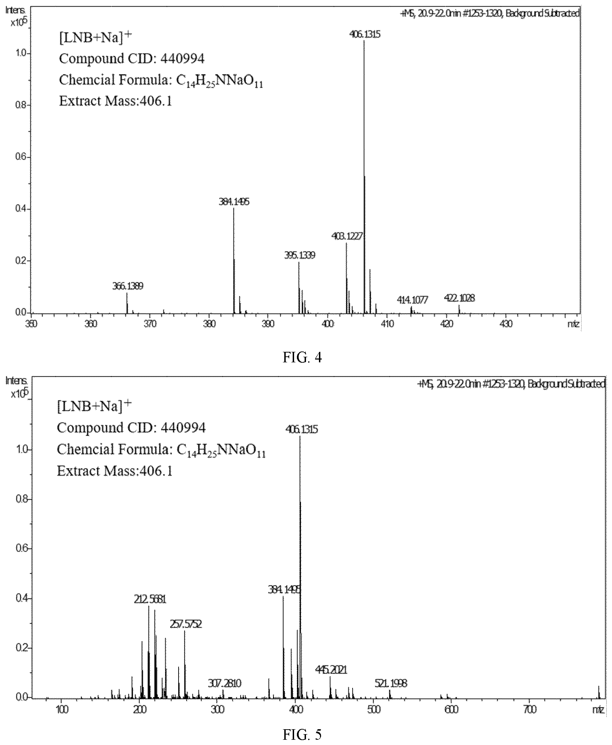

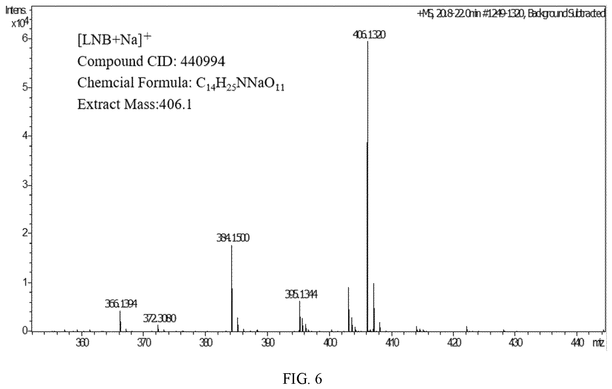

LC-MS graphs of the LNB standard substance and the substances with retention time of 10.3 min in the catalytic products of Bgap, Bgap-8 and Bgap-11 were respectively shown in FIG. 3, FIG. 4, FIG. 5 and FIG. 6.

As shown in an LC-MS graph, the molecular weight of LNB+H of the LNB standard substance was 384, and he molecular weight of LNB+Na was 406; therefore, in the LC-MS graph of substances with the retention time of 10.3 min in Bgap, Bgap-8 and Bgap-11 enzyme-catalytic products, the molecular weight of 384 was LNB+H, and the molecular weight of 406 was LNB+Na.

By searching the mass spectrum result, the following conclusions might be obtained: Compound CID: 440994, Chemcial Formula: C₁₄H₂₅NO₁₁, Extract Mass: 383.

In conclusion, the molecular weight of the product synthesized using galactose-1-phosphate (Gal-1-P) and N-acetylglucosamine as substrates under the catalytic action of Bgap-8 and Bgap-11 enzymes was about 383, and the retention time was consistent with that of the LNB standard substance, that is to say, the Bgap-8 and Bgap-11 enzyme catalytic reaction solution contained lacto-N-biose (LNB).

Further, according to the same method, the enzyme catalysis reaction solution of Bgap-1, Bgap-2, Bgap-3, Bgap-4, Bgap-5, Bgap-6, Bgap-7, Bgap-9, Bgap-10, Bgap-12 and Bgap-13 enzymes in Example 11 was analyzed through HPLC and LC-MS, and the same conclusion was obtained, namely, lacto-N-biose (LNB) was synthesized through these enzymes' catalysis, and only the yields of LNB were different, which were consistent with the content data of the generated LNB recorded in Example 11.

Therefore, it could be known that 1,3-β-galactosyl-N-acetylhexosamine phosphorylase (Bgap) and the mutant thereof catalyzed galactose-1-phosphate (Gal-1-P) and N-acetylglucosamine in the above reaction to synthesize lacto-N-biose (LNB with the molecular weight of 383).

### Example 13. Effect of temperature on catalytic synthesis of LNB by a glycoside hydrolase or mutants thereof

The effect of temperature on the reaction system was investigated in order to further improve the synthesis efficiency of LNB. The specific method was shown as follows:
0.3 mg/mL of Bgap, Bgap-1, Bgap-6, Bgap-7, Bgap-8, Bgap-10 and Bgap-11 glycoside hydrolase and 100 mM of Tris-HCl buffer solution were added into 32 mM of galactose-1-phosphate and 20 mM of N-acetylglucosamine, and the pH value was 7.0. The reaction was continuously performed at 10°C, 20°C, 30°C, 40°C, 50°C, 60°C and 70°C for 16 h, and then the reaction was terminated; the reaction solution was purified with a gel column method; and the concentration of LNB in the purified reaction solution was detected by HPLC, and the result is shown in Table 4.

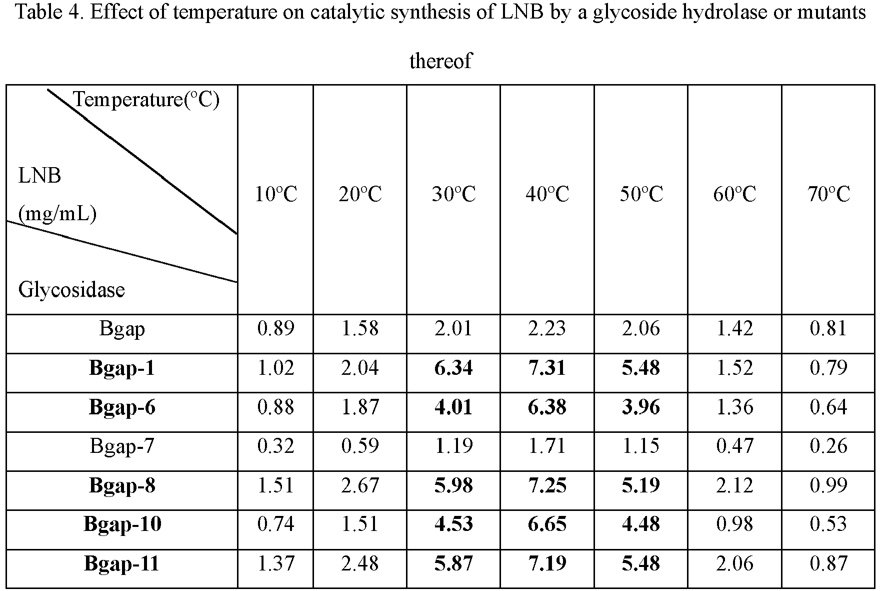

The result showed that the glycoside hydrolase had strong ability of catalyzing the synthesis of LNB within the temperature range of 30-50°C. The LNB synthesis efficiency was obviously reduced when the temperature exceeded 50°C. Presumably, the phenomenon may be related to the fact that the glycoside hydrolase was unstable and easily degenerated and inactivated at high temperature.

Moreover, as shown in Table 4, the LNB synthesis efficiency of the mutants Bgap-1, Bgap-6, Bgap-8, Bgap-10 and Bgap-11 is far higher than that of the original enzyme Bgap within the proper temperature range of 30-50°C.

### Example 14. Effect of pH value on catalytic synthesis of LNB by a glycoside hydrolase or mutants thereof

The effect of pH on the reaction system was investigated in order to further improve the synthesis efficiency of LNB. The specific method was shown as follows:
0.3 mg/mL of the glycoside hydrolase or mutants thereof and 100 mM of a Tris-HCl buffer solution were respectively added into 32 mM of galactose-1-phosphate and 20 mM of N-acetylglucosamine at 37°C. The reaction was continuously performed for 16 h under the pH value of 5.0, 6.0, 7.0, 8.0, 9.0, 10.0 and 11.0 respectively, and then the reaction was terminated; the reaction solution was purified by a gel column method; and the concentration of LNB in the purified reaction solution was detected by HPLC, and the results are shown in Table 5.

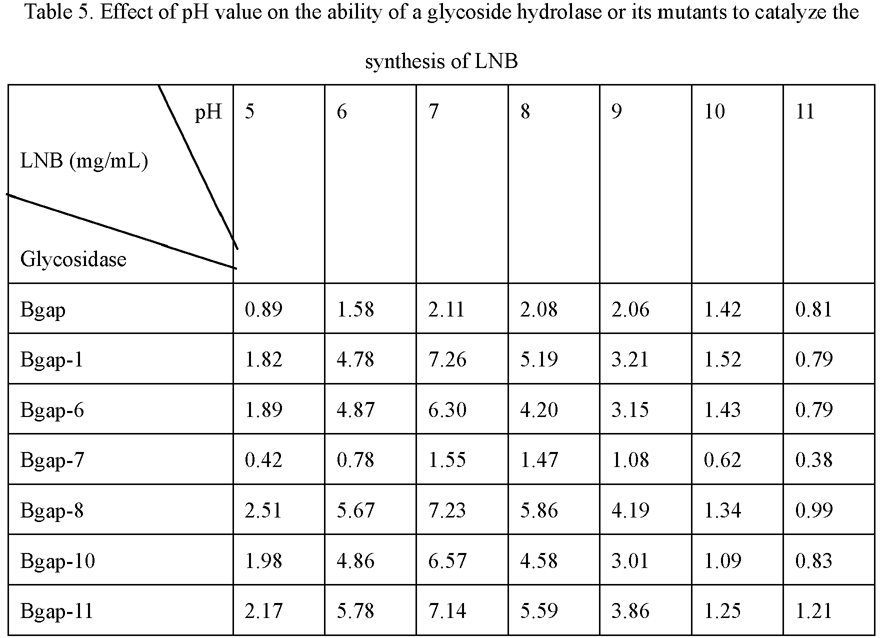

The result showed that glycoside hydrolase and mutants thereof had strong ability to catalyze the synthesis of LNB in the pH range of 6-8. Under strong alkaline or strong acidic conditions, the activity of the glycoside hydrolase and mutants thereof for synthesizing LNB was obviously reduced.

Moreover, as shown in Table 5, the LNB synthesis efficiency of the mutants Bgap-1, Bgap-6, Bgap-8, Bgap-10 and Bgap-11 is far higher than that of the original enzyme Bgap in the proper pH value range of pH 6-8.

### Example 15. Effect of reaction time on catalytic synthesis of LNB by a glycoside hydrolase or mutants thereof

0.3 mg/mL of the glycoside hydrolase or mutants thereof and 100 mM Tris-HCl buffer solution were respectively added to 32 mM of galactose-1-phosphate and 20 mM of N-acetylglucosamine, and reacted at 37 °C with pH value is 7.0 for 2 h, 4 h, 6 h, 12 h, 16 h, 20 h and 30 h, and then the reaction was terminated; the reaction solution was purified by a gel column method; and the LNB concentration in the purified solution was detected by HPLC, and the results are shown in Table 6.

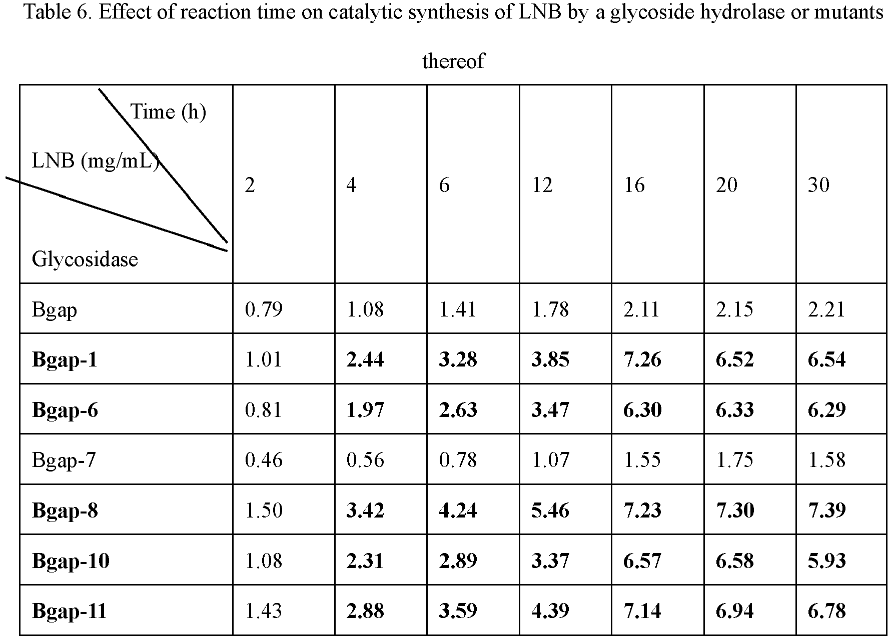

The result showed that a certain amount of LNB was generated until the reaction time reached 2 hours when glycoside hydrolase and its mutants are used to catalyze the synthesis of LNB; in the 4^{th}-16^{th} h, the concentration of LNB in a reaction solution was rapidly increased; after that the concentration of LNB in the reaction solution was slowly increased.

In addition, as shown in Table 6, after the 4^{th} h, the synthesis efficiency of LNB catalyzed by mutants Bgap-1, Bgap-6, Bgap-8, Bgap-10 and Bgap-11 was significantly higher than that of an original enzyme Bgap.

### Example 16. Effect of enzyme-added amount on catalytic synthesis of LNB by a glycoside hydrolase or mutants thereof

The effect of enzyme-added amount on the reaction system was investigated in order to further improve the synthesis efficiency of LNB. The specific method was shown as follows:
0.1 mg/mL, 0.2 mg/mL, 0.3 mg/mL, 0.5 mg/mL, 0.6 mg/mL, and 1.0 mg/mL of glycoside hydrolase or mutants thereof and 100 mM of Tris-HCl buffer solution were respectively added into 32 mM of galactose-1-phosphate and 20 mM of N-acetylglucosamine at 37°C with the pH value of 7.0; the mixture was reacted at 37°C for 16 h, and then the reaction was terminated; the reaction solution was purified by a gel column method; and the concentration of LNB in the purified solution was detected by HPLC, and the results are shown in Table 7.

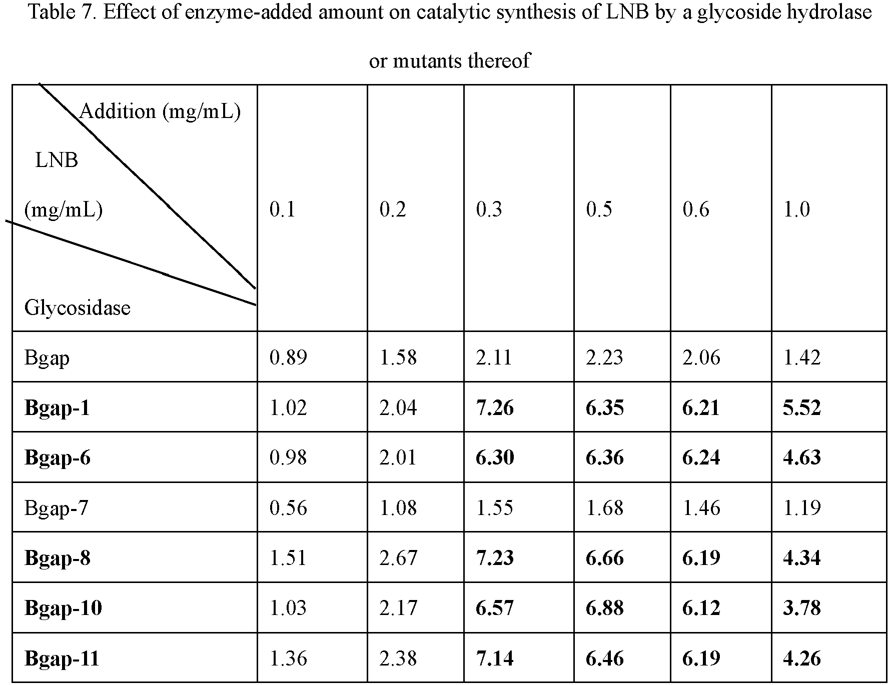

The result showed that the glycoside hydrolase and mutants thereof were used for catalytic synthesis of LNB, LNB was generated when the additive amount of enzyme was 0.1 mg/mL, the LNB generation amount was rapidly increased along with the increase of the enzyme, and the LNB generation was reduced after the additive amount was over 0.5 mg/mL.

In addition, as shown in Table 7, the LNB synthesis efficiency of mutants Bgap-1, Bgap-6, Bgap-8, Bgap-10 and Bgap-11 is higher than that of the original enzyme Bgap under each enzyme dosage; and particularly, after the additive amount was over 0.3 mg/mL, the LNB yields of the mutants Bgap-1, Bgap-6, Bgap-8, Bgap-10 and Bgap-11 are about 3 times or more higher than that of the original enzyme Bgap.

### Example 17. Research on catalytic synthesis of LNB by using lactose as glycosyl donor

A large amount of cheap raw material lactose could be used as the glycosyl donor. The efficiency of the catalytic synthesis of LNB by using glycoside hydrolase or mutants was detected. The specific method was as follows:
Lactose was added into 100 mM of Tris-HCl buffer solution (pH 7.0) at the final concentration of 40 mM; lactase was added according to the proportion of adding 120 U of lactase into every gram of lactose, reacted at 37°C for 5 h, and then boiled for deactivating enzyme;

Next, other components were added into the reaction solution, and the concentration of each component in the reaction solution was adjusted; the reaction system included: 20 mM of N-acetylglucosamine, 32 mM of ATP, 0.3 mg/mL of glycoside hydrolase, 0.13 mg/mL of galactokinase and 100 mM of Tris-HCl buffer solution at the pH of 7.0; the reaction was carried out at 37°C for 20 h, and then terminated; the reaction solution was purified by a gel column method; the concentration of LNB in the purified solution was detected by HPLC, and the transformation rate was calculated; and then results are shown in Table 8.

**Table 8. Research on catalytic synthesis of LNB by a glycoside hydrolase or mutants thereof**

| Original enzyme or mutants | Generated LNB (mg/mL) | GlcNAc Transformation rate (%) | Productivity (mg/mL/h) |
|---|---|---|---|
| Bgap | 1.64 | 21.41 | 0.08 |
| **Bgap-1** | **6.98** | **91.04** | **0.35** |
| Bgap-2 | 0.32 | 4.21 | 0.02 |
| Bgap-3 | 0.53 | 6.99 | 0.03 |
| Bgap-4 | 0.81 | 10.53 | 0.04 |
| Bgap-5 | 0.89 | 11.53 | 0.04 |
| **Bgap-6** | **5.78** | **75.39** | **0.29** |
| Bgap-7 | 1.20 | 15.73 | 0.06 |
| **Bgap-8** | **7.08** | **92.34** | **0.35** |
| Bgap-9 | 0.69 | 9.03 | 0.03 |
| **Bgap-10** | **6.48** | **84.52** | **0.32** |
| **Bgap-11** | **6.94** | **90.52** | **0.35** |
| Bgap-12 | 0.31 | 4.04 | 0.02 |
| Bgap-13 | 0.46 | 6.00 | 0.02 |

The result showed that under the condition of using lactose as a raw material, the glycoside hydrolase and mutants thereof can still catalyze the synthesis of LNB by reacting with other raw materials; moreover, the LNB yields, GlcNAc transformation rates and productivities of the mutants Bgap-1, Bgap-6, Bgap-8, Bgap-10 and Bgap-11 were far higher than those of the original enzyme Bgap.

Finally, it should be noted that the above examples are only used for illustrating the technical solution of the present application, but not to limit it; although the present application has been described in detail with reference to the foregoing examples, those of ordinary skill in the art should understand that modifications still can be made to the technical solutions described in the foregoing examples, or equivalent substitutions are made to some of the technical features; however, these modifications or substitutions do not cause the essence of the corresponding technical solutions to deviate from the spirit and scope of the technical solution of the present application.

### Industrial Applicability

According to the present application, the polypeptides with glycoside hydrolase activity efficiently catalyze acetylglucosamine or the carbohydrate with an acetylglucosamine group to generate lacto-N-biose (LNB) in the presence of the glycosyl donor. And moreover, the LNB production process in the present application is simple to operate; and compared with the prior art requiring reaction time of 600 h, the reaction time of the process in this application is decreased by about 30 times, therefore, the production efficiency is significantly improved, which is suitable for industrial production.

## Claims

1. A polypeptide with glycoside hydrolase activity, comprising an amino acid sequence selected from the follows:
the amino acid sequence obtained by modifying, substituting, deleting, or adding one or more amino acids of the amino acid sequence shown in SEQ ID NO: 1, which has glycoside hydrolase activity;
wherein the modification, substitution, deletion or addition of one or more amino acids is selected from the follows:
i) a combination of substitution from glutamic acid to glycine at position 229, substitution from valine to glycine at position 459, substitution from alanine to glycine at position 460, and substitution from alanine to leucine at position 271;
ii) a combination of substitution from aspartic acid to asparagine at position 233, substitution from glycine to valine at position 339, substitution from threonine to phenylalanine at position 343, and substitution from valine to glycine at position 459;
iii) a combination of substitution from aspartic acid to asparagine at position 233, substitution from alanine to leucine at position 271, substitution from glycinamide to serine at position 323, and substitution from glycine to valine at position 339;
iv) a combination of substitution from glutamic acid to glycine at position 229, substitution from alanine to leucine at position 271, substitution from glycine to valine at position 339, and substitution from valine to glycine at position 459; and
v) a combination of substitution from valine to glycine at position 459, and substitution from alanine to glycine at position 460.

2. A polynucleotide encoding the polypeptide according to claim 1.

3. A nucleic acid construct, comprising the polynucleotide according to claim 2, and one or more regulatory sequences operably linked to the polynucleotide, wherein the regulatory sequences can guide the expression of the polypeptide in an appropriate host.

4. An expression vector, comprising the polynucleotide according to claim 2, or the nucleic acid construct according to claim 3.

5. A host cell transformed with the nucleic acid construct according to claim 3 or the expression vector according to claim 4.

6. The transformed host cell according to claim 5, wherein the host cell is any one type of bacteria, fungi, or yeast;
preferably, the bacteria are selected from: *Escherichia coli, Vibrio natriegens, Bacillus subtilis, Bacillus licheniformis,* and *Bacillus amyloliquefaciens;*
preferably, the fungi are selected from: *Aspergillus oryzae, Aspergillus niger, Penicillium oxalicum,* and *Trichoderma reesei;* and
preferably, the yeast are selected from: *Pichia pastoris, Saccharomyces cerevisiαe, Kluyveromyces lactis*, and *Kluyveromyces marxianus.*

7. An enzyme agent or an enzyme composition, comprising the polypeptide according to claim 1 and optionally other enzyme;
preferably, the other enzyme is lactase and/or galactokinase.

8. A production method of the polypeptide according to claim 1, comprising:
(1) culturing a host cell containing a nucleic acid construct harboring a nucleotide sequence encoding the polypeptide, under conditions conducive to the production of the polypeptide; and
(2) collecting the polypeptide.

9. A method for producing lacto-N-biose, comprising: using a glycoside hydrolase as shown in SEQ ID NO: 1 or a polypeptide with glycoside hydrolase activity according to claim 1 as a catalyst, to catalyze the reaction of acetylglucosamine or a carbohydrate with an acetylglucosamine group with a glycosyl donor to generate lacto-N-biose;
preferably, the glycosyl donor is any one of or a combination of two or more of galactose-1-phosphate, lactose, and galactose;
preferably, the reaction is performed at 10-70°C, preferably 20-50°C, more preferably 30-50°C; preferably, the reaction is performed in a buffer solution with pH of 4-11, preferably pH of 6-8; and further preferably, the buffer solution is selected from:
a 3-morpholinopropanesulfonic acid (MOPS) buffer solution with a pH buffer range of 6.5-7.9;
a 4-(2-Hydroxyethyl)-1-piperazineethanesulfonic acid (HEPES) buffer solution with a pH buffer range of 6.8-8.2;
a tris(hydroxymethyl)aminomethane hydrochloride (Tris-HCl) buffer solution with a pH buffer range of 7.0-9.0; and
a phosphate buffer solution with a pH buffer range of 5.8-8.0.

10. Use of a glycoside hydrolase as shown in SEQ ID NO: 1 or a polypeptide according to claim 1 as a catalyst in production of human milk oligosaccharides, especially lacto-N-biose;
preferably, the production of lacto-N-biose is by the method according to claim 9.
